# EUROPEAN PATENT SPECIFICATION

(11) **EP 1 948 234 B1**
(45) Date of publication and mention of the grant of the patent: **05.12.2012**
(21) Application number: 06844226.8
(22) Date of filing: 27.10.2006
(51) Int. Cl.: A61K 39/395, C12P 21/08, C07K 16/00

(54) **COMPOSITIONS AND METHODS FOR THE TREATMENT AND PREVENTION OF FIBROTIC, INFLAMMATORY AND NEOVASCULARIZATION CONDITIONS**
ZUSAMMENSETZUNGEN UND VERFAHREN ZUR BEHANDLUNG UND PRÄVENTION VON FIBROTISCHEN, ENTZÜNDUNGS- UND NEOVASKULARISATIONSLEIDEN
COMPOSITIONS ET MÉTHODES DE TRAITEMENT ET DE PRÉVENTION DES PATHOLOGIES INFLAMMATOIRES, DE FIBROGENÈSE ET DE NÉOVASCULARISATION

(30) Priority: 28.10.2005 US 261935
(43) Date of publication of application: 30.07.2008
(73) Proprietor: Lpath, Inc., San Diego, CA 92121-3249 (US)
(72) Inventor: SABBADINI, Roger, A., Lakeside, CA 92040 (US); GARLAND, William, A., San Clemente, CA 92672 (US); STOLLER, Glenn, L., Roslyn, NY 11576 (US)
(74) Representative: Schiweck, Weinzierl & Koch
(86) International application number: PCT/US2006/042027
(87) International publication number: WO 2007/053447

(56) References cited:
- WO-A2-02/17899
- WO-A2-2006/078336
- US-B2- 6 858 383
- INVESTIGATIVE OPHTHALMOLOGY & VISUAL SCIENCE JUL 2002 LNKD- PUBMED:12091450, vol. 43, no. 7, July 2002 (2002-07), pages 2450-2461, XP002578897 ISSN: 0146-0404
- IOVS, vol. 46, no. Suppl. S, 2005, page 1319, XP009132466 ANNUAL MEETING OF THE ASSOCIATION-FOR-RESEARCH-IN-VISION-AND-OPH THALM OLOGY; FT LAUDERDALE, FL, USA; MAY 01 -05, 2005 ISSN: 0146-0404
- THE JOURNAL OF BIOLOGICAL CHEMISTRY 14 MAY 2004 LNKD- PUBMED:15023998, vol. 279, no. 20, 14 May 2004 (2004-05-14) , pages 20555-20558, XP002578899 ISSN: 0021-9258
- PYNE S ET AL: "REVIEW ARTICLE SPHINGOSINE 1-PHOSPHATE SIGNALLING IN MAMMALIAN CELLS" BIOCHEMICAL JOURNAL, THE BIOCHEMICAL SOCIETY, LONDON, GB LNKD- DOI:10.1042/0264-6021:3490385, vol. 349, 1 January 2000 (2000-01-01), pages 385-402, XP000939036 ISSN: 0264-6021
- EXPERIMENTAL EYE RESEARCH MAR 2009 LNKD- PUBMED:18723015, vol. 88, no. 3, March 2009 (2009-03), pages 367-377, XP002578900 ISSN: 1096-0007
- SVETLOV ET AL.: 'EDG receptors and hepatic Pathophysiology of LPA and SIP: EDG-ology of Liver Injury' BIOCHIMICA ET BIOPHYSICA ACTA vol. 1582, 2002, pages 251 - 256, XP004366547

## Description

### TECHNICAL FIELD

Disclosed herein are methods of treatments for ocular disorders using immune-derived moieties which are reactive against bioactive lipid molecules that play role in human and/or animal disease as signaling molecules. One particular class of signaling bioactive lipids considered in accordance with the present disclosure is lysolipids. Particularly preferred signaling lysolipids are sphingosine-1-phosphate (S1P) and the various lysophosphatidic acids (LPAs). Antibodies against signaling lipids, and derivatives and variants thereof, can be used in the treatment and/or prevention of ocular diseases or disorders through the delivery of pharmaceutical compositions that contain such antibodies, alone or in combination with other therapeutic agents and/or treatments.

### BACKGROUND OF THE INVENTION

### I. Introduction

The following description includes information that may be useful in understanding the present invention. It is not an admission that any such information is prior art, or relevant, to the presently claimed inventions, or that any publication specifically or implicitly referenced is prior art or even particularly relevant to the presently claimed invention.

### II. Background

The present invention relates to methods of decreasing or attenuating aberrant neovascularization, angiogenesis, aberrant fibrogenesis, fibrosis and scarring, and inflammation and immune responses. These processes, separately or together are involved in many diseases and conditions. These diseases or conditions may be systemic or may be relatively localized, for example to the skin or to the eye.

### A. Ocular diseases and conditions

Pathologic or aberrant angiogenesis/neovascularization, aberrant remodeling, fibrosis and scarring and inflammation occur in association with retinal and ocular ischemic diseases such as age-related macular degeneration (AMD), diabetic retinopathy (DR) and in retinopathy of prematurity (ROP) and other developmental disorders [Eichler et al. (2006), Curr Pharm Des, vol 12: 2645-60] as well as being a result of infections and mechanical injury to the eye [Ciulla et al. (2001), Curr Opin Ophthalmol, vol 12: 442-9 and Dart et al (2003), Eye, vol 17: 886-92].

Pathologic ocular angiogenesis is a leading cause of blindness in a variety of clinical conditions. Choroidal neovascularization (CNV) occurs in a number of ocular diseases, the most prevalent of which is the exudative or "wet" form of AMD. As a result of an increasingly aged population, AMD is a modem day epidemic and the leading cause of blindness in the western world in patients over age 60. Despite the epidemic of vision loss caused by AMD, only a few therapies, mostly anti-VEGF based, can slow the progression of AMD and even fewer can reverse vision loss [Bylsma and Guymer (2005), Clin Exp Optom,. vol 88: 322-34, Gryziewicz (2005), Adv Drug Deliv Rev, vol 57: 2092-8 and Liu and Regillo (2004), Curr Opin Ophthalmol, vol 15: 221-6.]. Therefore, discovering new treatments for pathologic neovascularization is extremely important.

AMD is used here solely for illustrative purposes in describing ocular conditions relating to aberrant angiogenesis/neovascularization, aberrant remodeling, fibrosis and scarring, and inflammation, which conditions are found in other ocular diseases and disorders as disclosed and claimed herein. AMD involves age-related pathologic changes [Tezel, Bora and Kaplan (2004), Trends Mol Med, vol 10: 417-20 and Zarbin (2004), Arch Ophthalmol, 122: 598-614]. Multiple theories exist but, the exact etiology and pathogenesis of AMD are still not well understood. Aging is associated with cumulative oxidative injury, thickening of Bruch's membrane and drusen formation. Oxidative stress results in injury to retinal pigment epithelial (RPE) cells and, in some cases, the choriocapillaris [Zarbin (2004), Arch Ophthalmol, vol 122: 598-614 and Gorin et al. (1999), Mol Vis,. vol 5: 29]. Injury to RPE likely elicits a chronic inflammatory response within Bruchs membrane and the choroid [Johnson et al. (2000), Exp Eye Res,. vol 70: 441-9]. This injury and inflammation fosters and potentates retinal damage by stimulating CNV and atrophy [Zarbin (2004), Arch Ophthalmol, vol 122: 598-614 and Witmer et al. (2003), Prog Retin Eye Res, vol 22: 1-29]. CNV results in defective and leaky blood vessels (BV) that are likely to be recognized as a wound [Kent and Sheridan (2003), Mol Vis, vol 9: 747-55]. Wound healing arises from the choroid and invades the subretinal space through Bruchs membrane and the RPE. Wound healing responses are characterized by a typical early inflammation response, a prominent angiogenic response and tissue formation followed by end-stage maturation of all involved elements. Wound remodeling may irreversibly compromise photoreceptors and RPEs thereby, justifying the need to treat CNV with more than anti-angiogenic therapies [La Cour, Kiilgaard and Nissen (2002), Drugs Aging, vol 19: 101-33.12].

Alterations in the normal retinal and sub-retinal architecture as a result of CNV related fibrosis, edema and inflammation individually or cumulatively, leads to AMD related visual loss [Tezel and Kaplan (2004), Trends Mol Med, vol 10: 417-20 and Ambati et al. (2003), Surv Ophthalmol, vol 48: 257-93]. The multiple cellular and cytokine interactions which are associated with exudative AMD greatly complicate the search for effective treatments. While CNV and edema are manageable in part by anti-VEGF therapeutics, potential treatments to mitigate scar formation and inflammation have not been adequately addressed [Bylsma and Guymer (2005), Clin Exp Optom, vol 88: 322-34 and Pauleikhoff (2005), Retina, vol 25: 1065-84]. As long as the neovascular complex remains intact, as appears to be the case in patients treated with anti-VEGF agents, the potential for subretinal fibrosis and future vision loss persists.

Anti-VEGF-A therapies represent a recent, significant advance in the treatment of exudative AMD. However, the phase III VISION Trial with PEGAPTANIB, a high affinity aptamer which selectively inhibits the 165 isoform of VEGF-A, demonstrated that the average patient continues to lose vision and only a small percent gained vision [Gragoudas et al. (2004), N Engl J Med, vol 351: 2805-16]. Inhibition of all isoforms of VEGF-A (pan-VEGF inhibition) with the antibody fragment RANIBIZUMAB yielded much more impressive results [Brown et al. N Eng Med,2006 355:1432-44, Rosenfeld et al. N Eng J Med 2006355:1419-31]. The 2 year MARINA trial and the 1 year ANCHOR trial demonstrated that approximately 40% of patients achieve some visual gain. Although these results represent a major advance in our ability to treat exudative AMD, they also demonstrate that 60% of patients do not have visual improvement. Furthermore, these patients had to meet strictly defined inclusion and exclusion criteria. The results in a larger patient population may be less robust.

There is still a well defined need to develop further therapeutic agents that target other steps in the development of CNV and the processes that ultimately lead to photoreceptor destruction. First, the growth of choroidal BVs involves an orchestrated interaction among many mediators, not just VEGF, offering an opportunity to modulate or inhibit the entire process [Gragoudas et al. (2004), N Engl J Med, vol 351: 2805-16]. Second, exudative AMD is comprised of vascular and extravascular components. The vascular component involves vascular endothelial cells (EC), EC precursors and pericytes. The extravascular component, which volumetrically appears to be the largest component, is composed of inflammatory, glial and retinal pigment epithelium (RPE) cells and fibroblasts. Tissue damage can result from either component. These other aspects of the pathologic process are not addressed by current anti-VEGF treatments. Targeting additional elements of the angiogenic cascade associated with AMD could provide a more effective and synergistic approach to therapy [Spaide RF (2006), Am J Ophthalmol, vol 141: 149-156].

### 1. Inflammation in ocular disease

There is increasing evidence that inflammation, specifically macrophages and the complement system [Klein et al. (2005), Science, vol 308: 385-9 and Hageman et al. (2005), Proc Natl Acad Sci U S A, vol 102: 7227-32] play an important role in the pathogenesis of exudative AMD. Histopathology of surgically excised choroidal neovascular membranes demonstrates that macrophages are almost universally present [Grossniklaus, et al. (1994), Ophthalmology, vol 101: 1099-111 and Grossniklaus et al. (2002), Mol Vis, vol 8: 119-26]. There is mounting evidence that macrophages may play an active role in mediating CNV formation and propagation [Grossniklaus et al. (2003), Mol Vis, vol 8: 119-26; Espinosa-Heidmann, et al. (2003), Invest Ophthalmol Vis Sci, vol 44: 3586-92; Oh et al. (1999), Invest Ophthalmol Vis Sci, vol 40: 1891-8; Cousins et al. (2004), Arch Ophthalmol, vol 122: 1013-8; Forrester (2003), Nat Med, vol 9: 1350-1 and Tsutsumi et al. (2003), J Leukoc Biol, vol 74: 25-32] by multiple effects which include secretion of enzymes that can damage cells and degrade Bruchs membrane as well as release pro-angiogenic cytokines [Otani et al. (1999), Ophthalmol Vis Sci, vol 40: 1912-20 and Amin, Puklin and Frank (1994), Invest Ophthalmol Vis Sci, vol 35: 3178-88] At the site of injury, macrophages exhibit micro-morphological signs of activation, such as degranulation [Oh et al. (1999), Invest Ophthalmol Vis Sci, vol 40: 1891-8 and Trautmann et al. (2000), J Pathol, vol 190: 100-6]. Thus it is believed that a molecule which limited macrophage infiltration into to the choroidal neovascular complex may help limit CNV formation.

### 2. Choroidal neovascularization and blood vessel maturation in ocular disease

Angiogenesis is an essential component of normal wound healing as it delivers oxygen and nutrients to inflammatory cells and assists in debris removal [Lingen (2001), Arch Pathol Lab Med, vol 125: 67-71]. Progressive angiogenesis is composed of two distinct processes: Stage I: Migration of vascular ECs, in response to nearby stimuli, to the tips of the capillaries where they proliferate and form luminal structures; and Stage II: Pruning of the vessel network and optimization of the vasculature [Guo et al. (2003), Am J Pathol, vol 162: 1083-93].

Stage I: Neovascularization. Angiogenesis most often aids wound healing. However, new vessels when uncontrolled, are commonly defective and promote leakage, hemorrhaging and inflammation. Diminishing dysfunctional and leaky BVs, by targeting pro-angiogenic GFs, has demonstrated some ability to slow the progression of AMD [Pauleikhoff (2005), Retina, vol 25: 1065-84.14 and van Wijngaarden, Coster and Williams (2005), JAMA, vol 293: 1509-13].

Stage II: Blood vessel maturation and drug desensitization. Pan-VEGF inhibition appears to exert its beneficial effect mostly via an anti-permeability action resulting in resolution of intra- and sub-retinal edema, as the actual CNV lesion does not markedly involute [Presentation. at Angiogenesis 2006 Meeting. 2006. Bascom Palmer Eye Institute Miami, Florida]. The lack of marked CNV involution may in part be a result of maturation of the newly formed vessels due to pericyte coverage. Pericytes play a critical role in the development and maintenance of vascular tissue. The presence of pericytes seems to confer a resistance to anti-VEGF agents and compromise their ability to inhibit angiogenesis [Bergers and Song (2005), Neuro-oncol, vol 7: 452-64; Yamagishi and Imaizumi (2005), Int J Tissue React, vol 27: 125-35; Armulik, Abramsson and Betsholtz (2005), Circ Res, vol 97: 512-23; Ishibashi et al. (1995), Arch Ophthalmol, vol 113: 227-31]. An agent which has an inhibitory effect on pericyte recruitment would likely disrupt vascular channel assembly and the maturation of the choroidal neovascular channels thereby perpetuating their sensitivity to anti-angiogenic agents.

Remodeling of the vascular network involves adjustments in BV density to meet nutritional needs [Gariano and Gardner (2005), Nature, 438: 960-6]. Periods of BV immaturity corresponds to a period in which new vessels are functioning but have not yet acquired a pericyte coating [Benjamin, Hemo and Keshet (1998), Development, 125: 1591-8 and Gerhardt and Betsholtz (2003), Cell Tissue Res, 2003. 314: 15-23]. This delay is essential in providing a window of plasticity for the fine tuning of the developing vasculature according to the nutritional needs of the retina or choroid.

The bioactive lipid sphingosine-1-phosphate (S1P), VEGF, PDGF, angiopoietins (Ang) and other growth factors (GF) augment blood vessel growth and recruit smooth muscle cells (SMC) and pericytes to naive vessels which promote the remodeling of emerging vessels [Allende and Proia (2002), Biochim Biophys Acta, vol 582: 222-7; Gariano and Gardner (2005), Nature, vol 438: 960-6; Grosskreutz et al. (1999), Microvasc Res, vol 58: 128-36; Nishishita, and Lin (2004), J Cell Biochem, vol 91: 584-93 and Erber et al. (2004), FASEB J, vol 18: 338-40.32]. Pericytes, most likely generated by in situ differentiation of mesenchymal precursors at the time of EC sprouting or from the migration and de-differentiation of arterial smooth muscle cells, intimately associate and ensheath ECs resulting in overall vascular maturity and survival [Benjamin, Hemo and Keshet (1998), Development, vol 125: 1591-8]. Recent studies have demonstrated that S1P, and the S1P1 receptor, are involved in cell-surface trafficking and activation of the cell-cell adhesion molecule N-cadherin [Paik et al. (2004), Genes Dev, vol 18: 2392-403]. N-cadherin is essential for interactions between EC, pericytes and mural cells which promote the development of a stable vascular bed [Gerhardt and Betsholtz (2003), Cell Tissue Res, vol 314: 15-23]. Global deletion of the S1P1 gene results in aberrant mural cell ensheathment of nascent BVs required for BV stabilization during embryonic development [Allende and Proia (2002), Biochim Biophys Acta, vol 1582: 222-7]. Local injection of siRNA to S1P1 suppresses vascular stabilization in tumor xenograft models [Chae et al. (2004), J Clin Invest, vol 114: 1082-9]. Transgenic mouse studies have demonstrated that VEGF and PDGF-B promote the maturation and stabilization of new BVs [Guo et al. (2003), Am J Pathol, 162: 1083-93 and Gariano and Gardner (2005), Nature, vol 438: 960-6.50]. VEGF up-regulates Ang-1 (mRNA and protein) [Asahara et al. (1998), Circ Res, vol 83: 233-40]. Ang-1 plays a major role in recruiting and sustaining peri-endothelial support by pericytes [Asahara et al. (1998), Circ Res, vol 83: 233-40]. Intraocular injection of VEGF accelerated pericyte coverage of the EC plexus [Benjamin, Hemo and Keshet (1998), Development, vol 125: 1591-8]. PDGF-B deficient mouse embryos lack micro-vascular pericytes, which leads to edema, micro-aneurisms and lethal hemorrhages [Lindahl et al. (1997), Science, vol 277: 242-5]. Murine pre-natal studies have demonstrated that additional signals are required for complete VEGF- and PDGF-stimulation of vascular bed maturation. Based upon the trans-activation of S1P noted above, this factor could be S1P [Erber et al. (2004), FASEB J, vol 18: 338-40]. Vessel stabilization and maturation is associated with a loss of plasticity and the absence of regression to VEGF and other GF withdrawal and resistance to anti-angiogenic therapies [Erber et al. (2004), FASEB J, vol 18: 338-40 and Hughes. and Chan-Ling (2004), Invest Ophthalmol Vis Sci, vol 45: 2795-806]. Resistance of BVs to angiogenic inhibitors is conferred by pericytes that initially stabilize matured vessels and those that are recruited to immature vessels upon therapy [Erber et al. (2004), FASEB J, vol 18: 338-40]. After ensheathment of the immature ECs, the pericytes express compensatory survival factors (Ang-1 and PDGF-B) that protect ECs from pro-apoptotic agents.

### 3. Edema and vascular permeability

CNV membranes are composed of fenestrated vascular ECs that tend to leak their intravascular contents into the surrounding space resulting in subretinal hemorrhage, exudates and fluid accumulation [Gerhardt and Betsholtz (2003), Cell Tissue Res, vol 14: 15-23]. For many years the CNV tissue itself, and more recently intra-retinal neovascularization, have been implicated as being responsible for the decrease in visual acuity associated with AMD. It is now thought however, that macular edema caused by an increase in vascular permeability (VP) and subsequent breakdown of the blood retinal barrier (BRB), plays a major role in vision loss associated with AMD and other ocular diseases [Hughes and Chan-Ling (2004), Invest Ophthalmol Vis Sci, vol 45: 2795-806; Felinski and Antonetti (2005), Curr Eye Res, vol 30: 949-57; Joussen et al. (2003), FASEB J, vol 17: 76-8 and Strom et al. (2005), Invest Ophthalmol Vis Sci, vol 46: 3855-8].

### 4. Fibrosis, fibrogenesis and scar formation

The formation of subretinal fibrosis leads to irreversible damage to the photoreceptors and permanent vision loss. As long as the neovascular complex remains intact, as appears to be the case in patients treated with anti-VEGF agents, the potential for subretinal fibrosis and future vision loss persists. In an update of the PRONTO study of RANIBIZUMAB, it was discovered that those patients who lost vision did so as a result of either subretinal fibrosis or a RPE tear [Presentation. at Angiogenesis 2006 Meeting. 2006. Bascom Palmer Eye Institute Miami, Florida.]. An agent that could diminish the degree of fibroblast infiltration and collagen deposition would likely be of value.

Fibroblasts, particularly myofibroblasts, are key cellular elements in scar formation in response to cellular injury and inflammation [Tomasek et al. (2002), Nat Rev Mol Cell Biol, vol 3: 349-63 and Virag and Murry (2003), Am J Pathol, vol 163: 2433-40]. Collagen gene expression by myofibroblasts is a hallmark of remodeling and necessary for scar formation [Sun and Weber (2000), Cardiovasc Res, vol 46: 250-6 and Sun and Weber (1996), J Mol Cell Cardiol, vol 28: 851-8]. S1P promotes wound healing by activating fibroblast migration and proliferation while increasing collagen production [Sun et al. (1994), J Biol Chem, vol 269: 16512-7]. S1P produced locally by damaged cells could be responsible for the maladaptive wound healing associated with remodeling and scar formation. Thus it is believed that S1P inhibitors are useful in diseases or conditions characterized, at least in part, by aberrant fibrogenesis or fibrosis. Herein, "fibrogenesis" is defined as excessive activity or number of fibroblasts, and "fibrosis" is defined as excessive activity or number of fibroblasts that leads to excessive or inappropriate collagen production and scarring, destruction of the physiological tissue structure and/or inappropriate contraction of the matrix leading to such pathologies as retinal detachment or other processes leading to impairment of organ function.

### B. Other diseases or conditions

The role of bioactive signaling lipids such as S1P and LPA is not limited to ocular diseases and conditions. Because of the involvement of biolipid signaling in many processes, including neovascularization, angiogenesis, aberrant fibrogenesis, fibrosis and scarring, and inflammation and immune responses, it is believed that antibody-based inhibitors of these bioactive lipids will be helpful in a variety of diseases and conditions associated with one or more of these processes. Such diseases and conditions may be systemic (e.g., systemic scleroderma) or localized to one or more specific body parts or organs (e.g., skin, lung, or eye).

### C. Bioactive signaling lipids

Lipids and their derivatives are now recognized as important targets for medical research, not as just simple structural elements in cell membranes or as a source of energy for β-oxidation, glycolysis or other metabolic processes. In particular, certain bioactive lipids function as signaling mediators important in animal and human disease. Although most of the lipids of the plasma membrane play an exclusively structural role, a small proportion of them are involved in relaying extracellular stimuli into cells. "Lipid signaling" refers to any of a number of cellular signal transduction pathways that use cell membrane lipids as second messengers, as well as referring to direct interaction of a lipid signaling molecule with its own specific receptor. Lipid signaling pathways are activated by a variety of extracellular stimuli, ranging from growth factors to inflammatory cytokines, and regulate cell fate decisions such as apoptosis, differentiation and proliferation. Research into bioactive lipid signaling is an area of intense scientific investigation as more and more bioactive lipids are identified and their actions characterized.

Examples of bioactive lipids include the eicosanoids (including the cannabinoids, leukotrienes, prostaglandins, lipoxins, epoxyeicosatrienoic acids, and isoeicosanoids), non-eicosanoid cannabinoid mediators, phospholipids and their derivatives such as phosphatidic acid (PA) and phosphatidylglycerol (PG), platelet activating factor (PAF) and cardiolipins as well as lysophospholipids such as lysophosphatidyl choline (LPC) and various lysophosphatidic acids (LPA). Bioactive signaling lipid mediators also include the sphingolipids such as sphingomyelin, ceramide, ceramide-1-phosphate, sphingosine, sphingosylphosphoryl choline, sphinganine, sphinganine-1-phosphate (Dihydro-S1P) and sphingosine-1-phosphate. Sphingolipids and their derivatives represent a group of extracellular and intracellular signaling molecules with pleiotropic effects on important cellular processes. Other examples of bioactive signaling lipids include phosphatidylserine (PS), phosphatidylinositol (PI), phosphatidylethanolamine (PEA), diacylglyceride (DG), sulfatides, gangliosides, and cerebrosides.

### D. Lysolipids

Lysophospholipids (LPLs), also known as lysolipids, are low molecular weight (typically less than about 500 dalton) lipids that contain a single hydrocarbon backbone and a polar head group containing a phosphate group. Some lysolipids are bioactive signaling lipids. Two particular examples of medically important bioactive lysolipids are LPA (glycerol backbone) and S1P (sphingoid backbone). The structures of selected LPAs, S1P, and dihydro S1P are presented below.

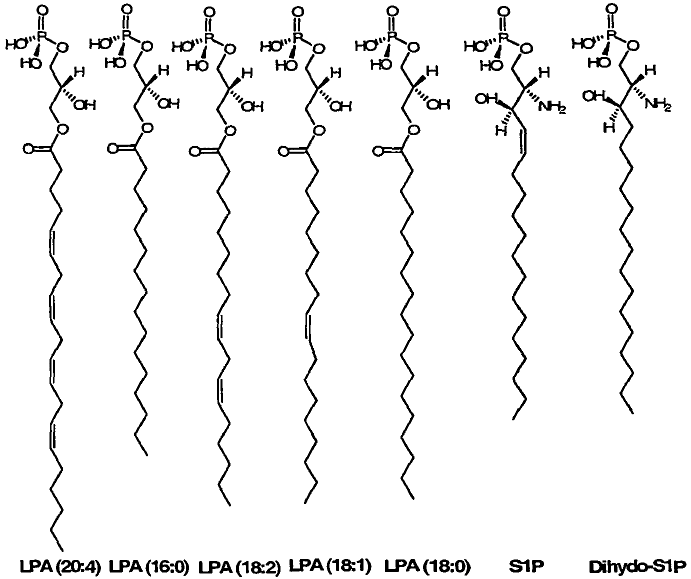

LPA is not a single molecular entity but a collection of endogenous structural variants with fatty acids of varied lengths and degrees of saturation (Fujiwara et al (2005), J Biol Chem, vol. 280: 35038-35050). The structural backbone of the LPAs is derived from glycerol-based phospholipids such as phosphatidylcholine (PC) or phosphatidic acid (PA). In the case of lysosphingolipids such as S1P, the fatty acid of the ceramide backbone is missing. The structural backbone of S1P, dihydro S1P (DHS1P), and sphingosylphosphorylcholine (SPC) is based on sphingosine, which is derived from sphingomyelin.

LPA and S1P regulate various cellular signaling pathways by binding to the same class of multiple transmembrane domain G protein-coupled (GPCR) receptors (Chun J, Rosen H (2006), Current Pharm Des, vol. 12: 161-171 and Moolenaar WH (1999), Experimental Cell Research, vol. 253: 230-238). The S1P receptors are designated as S1P₁, S1P₂, S1P₃, S1P₄ and S1P₅ (formerly EDG-1, EDG-5/AGR16, EDG-3, EDG-6 and EDG-8) and the LPA receptors designated as LPA₁, LPA₂, LPA₃ (formerly, EDG-2, EDG-4, and EDG-7). A fourth LPA receptor of this family has been identified for LPA (LPA₄), and other putative receptors for these lysophospholipids have also been reported.

### E. Sphingosine-1-phosphate

S1P is a mediator of cell proliferation and protects from apoptosis through the activation of survival pathways (Maceyka et al. (2002), BBA, vol 1585): 192-201 and Spiegel S. et al. (2003), Nature Reviews Molecular Cell Biology, vol 4: 397-407). It has been proposed that the balance between ceramide/sphingosine (CER/SPH) levels and S1P provides a rheostat mechanism that decides whether a cell is directed into the death pathway or is protected from apoptosis. The key regulatory enzyme of the rheostat mechanism is sphingosine kinase (SPHK) whose role is to convert the death-promoting bioactive signaling lipids (CER/SPH) into the growth-promoting S1P. S1P has two fates: S1P can be degraded by S1P lyase, an enzyme that cleaves S1P to phosphoethanolamine and hexadecanal, or, less common, hydrolyzed by S1P phosphatase to SPH. S1P is abundantly generated and stored in platelets, which contain high levels of SPHK and lacks the enzymes for S1P degradation. When platelets are activated, S1P is secreted. In addition, other cell types, for example, mast cells, are also believed to be capable of secreting S1P. Once secreted, S1P is thought to be bound at high concentrations on carrier proteins such as serum albumin and lipoproteins. S1P is found in high concentrations in plasma, with concentrations in the range of 0.5 - 5 uM having been reported. Though primarily extracellular, intracellular actions of S1P have also been suggested (see, eg, Spiegel S, Kolesnick R (2002), Leukemia, vol. 16: 1596-602; Suomalainen, et al (2005), Am J Pathol, vol. 166: 773-81).

Widespread expression of the cell surface S1P receptors allows S1P to influence a diverse spectrum of cellular responses, including proliferation, adhesion, contraction, motility, morphogenesis, differentiation, and survival. This spectrum of response appears to depend upon the overlapping or distinct expression patterns of the S1P receptors within the cell and tissue systems. In addition, crosstalk between S1P and growth factor signaling pathways, including platelet-derived growth factor (PDGF), vascular endothelial growth factor (VEGF), transforming growth factor beta (TGFβ) and basic fibroblastic growth factor (bFGF), have recently been demonstrated (see, e.g., Baudhuin, et al (2004), FASEB J, vol. 18: 341-3). Because regulation of various cellular processes involving S1P has particular impact on neuronal signaling, vascular tone, wound healing, immune cell trafficking, reproduction, and cardiovascular function, among others, it is believed that alterations of endogenous levels of S1P within these systems can have detrimental effects, eliciting several pathophysiologic conditions, including cancer, heart failure, ocular disease and infectious and autoimmune diseases. We propose that a potentially effective strategy for treating CNV associated with AMD is to reduce the biologically available extracellular levels of S1P. The applicants have developed a murine monoclonal antibody (SPHINGOMAB™, anti-S1P mAb) that is specific for S1P. SPHINGOMAB represents the first successfully created monoclonal antibody against a bioactive signaling sphingolipid target. SPHINGOMAB acts as a molecular sponge to selectively absorb S1P from the extracellular fluid, lowering the effective concentration of S1P. It selectively binds and neutralizes S1P with picomolar affinity in biologic matrices. We propose that SPHINGOMAB would deprive fibroblasts, pericytes, and endothelial, inflammatory and immune cells in the eye of important growth and survival factors thus targeting the multiple maladaptive steps of AMD resulting in the loss of photoreceptors and visual acuity. A therapeutic that simultaneously targets multiple components of the choroidal neovascular response has the potential to be a more potent therapeutic than "single-target" therapeutics.

As used herein, "sphingosine-1-phosphate" or "S1P" refers to sphingosine-1-phosphate [sphingene-1-phosphate; D-erythro-sphingosine-1-phosphate; sphing-4-enine-1-phosphate; (E,2S,3R)-2-amino-3-hydroxy-octadec-4-enoxy]phosphonic acid; CAS 26993-30-6] and its variants, S1P and DHS1P (dihydro sphingosine-1-phosphate [sphinganine-1-phosphate; [(2S,3R)-2-amino-3-hydroxy-octadecoxy]phosphonic acid; D-Erythro-dihydro-D-sphingosine-1-phosphate;CAS 19794-97-9] and sphingosylphosphorylcholine. "Variants" of S1P and LPA, as used herein, includes analogs and derivatives of S1P and LPA, respectively, which function similarly, or might be expected to function similarly, to the parent molecule.

Growing evidence suggests that S1P could contribute to both the early and late stages of maladaptive retinal remodeling associated with exudative AMD. S1P has a pronounced non-VEGF dependent pro-angiogenic effect. S1P also stimulates migration, proliferation and survival of multiple cell types, including fibroblasts, EC, pericytes and inflammatory cells-the same cells that participate in the multiple maladaptive processes of exudative AMD. S1P is linked to the production and activation of VEGF, bFGF, PDGF and other growth factors (GFs) implicated in the pathogenesis of exudative AMD. Finally, S1P may modulate the maturation of naive vasculature, a process leading to a loss of sensitivity to anti-angiogenic agents. Inhibiting the action of S1P could be an effective therapeutic treatment for exudative AMD that may offer significant advantages over exclusively anti-VEGF approaches or may act synergistically with them to address the complex processes and multiple steps that ultimately lead to AMD associated visual loss.

There is growing evidence that S1P is an important mediator of inflammatory events [Olivera and Rivera (2005), J Immunol,. vol 174: 1153-8]. Activated platelets, neutrophils, macrophages and mast cells serve as rich sources of S1P after coagulation and inflammatory events [Yatomi et al. (2000) Blood, vol 96: 3431-8]. Because these cells are important components in the inflammation response and tissue loss, S1P may regulate these events via control of inflammatory cell function [Tezel (2004), Trends Mol Med, vol 10: 417-20]. S1P released from mast cells is responsible for many of the responses in experimental animal models of inflammation [Jolly et al. (2004), J Exp Med,.vol 199: 959-70 and Jolly et al. (2005), Blood, vol 105: 4736-42]. Neutralizing S1P with SPHINGOMAB could provide an effective, novel means of limiting the deleterious inflammatory response that exacerbates ocular tissue damage of CNV associated with AMD.

Several lines of evidence suggest that S1P, and S1P's complement of receptors, may play a major regulatory role in the angiogenic process [Allende and Proia 2002), Biochim Biophys Acta,. vol 1582: 222-7; Spiegel (1993), J. Lipid Med,.vol 8: 169-175 and Argraves et al. (2004), J Biol Chem, vol 279: 50580-90]. First, S1P stimulates DNA synthesis and chemotactic motility of local and bone marrow-derived vascular EC to sites of vascularization, while inducing differentiation of multicellular structures consistent with early BV formation [Lee et al. (1999), Biochem Biophys Res Commun, vol 264: 743-325 and Annabi, et al (2003), Exp Hematology,. vol 31: 640-649]. Second, S1P stimulates the formation and maintenance of vascular EC assembly and integrity by activating both S1P₁ and S1P₃, and S1P-induced EC adherent junction assembly [Paik et al. (2004), Genes Dev, vol 18: 2392-403 and Lee et al. (1999), Cell, vol 99: 301-12]. Antisense oligonucleotides against these S1P receptors diminish S1P-induced vascular EC assembly and cell barrier integrity [English, et al. (1999), J Hematother Stem Cell Res, vol 8: 627-34 and Lee et al. (2001), Mol Cell, vol 8: 693-704]. Third, capillary tube formation induced by S1P has been demonstrated to be a more potent pro-angiogenic stimulus than bFGF or VEGF [Wang et al. (1999), J. Biol. Chem., vol 274: 35343-50 and Lee et al. (1999), Biochem Biophys Res Commun, vol 264: 743-325]. Finally, it has been shown that S1P elicits a synergic effect with VEGF, EGF, PDGF, bFGF and IL-8 to promote the development of vascular networks in vivo [Wang et al. (1999), J Biol. Chem., vol 274: 35343-50]. S1P trans-activates EGF and VEGF2 receptors [Tanimoto, Jin and Berk (2002), J Biol Chem, vol 277: 42997-3001] and VEGF up-regulates S1P receptors [Igarashi et al. (2003), Proc Natl Acad Sci U S A, vol 100: 10664-9]. Treatment of vascular ECs with VEGF markedly induces the up-regulation of S1P1 expression and enhances S1P-mediated signaling pathways leading to the activation of the endothelial isoform of nitric oxide synthase (eNOS) [Lee et al. (2001), Mol Cell, vol 8: 693-704 and Tanimoto, Jin and Berk (2002), J Biol Chem, vol 277: 42997-3001 and Igarashi and Michel (2001), J Biol Chem, vol 276: 36281-8]. eNOS activity plays a crucial role in different cellular responses and essential vascular functions, including inhibition of apoptosis, inhibition of platelet aggregation and angiogenesis [Kwon et al. (2001), J Biol Chem, vol 276: 10627-33; Huang (2003), Curr Hypertens Rep, vol 5: 473-80; Dantas, Igarashi Michel (2003), Am J Physiol Heart Circ Physiol, vol 284: H2045-52; Rkitake et al. (2002), Arterioscler Thromb Vasc Biol, vol 22: 08-114 and Kimura and Esumi (2003), Acta Biochim Pol, vol 50: 49-59]. Vascular structures resulting from the exposure to both bFGF and S1P were more differentiated that those obtained from the exposure to bFGF alone suggesting that S1P may be required for the full activity of bFGF and VEGF [English et al. (2000), FASEB J, vol 14: 2255-65.].

Thus, SPHINGOMAB may mitigate aberrant BV growth by neutralizing synergistic pro-angiogenic GFs and possibly S1P produced in excess during metabolic stress from inflammatory cells associated with CNV. SPHINGOMAB not only inhibits S1P-induced EC migration/infiltration and BV formation, but it also neutralizes bFGF and VEGF-induced vascularization through its effect on S1P. SPHINGOMAB has a potential advantage over "single-target" therapeutics because of its ability to neutralize S1P, which results in neutralization of multiple GFs via the pleiotropic effects of S1P.

Direct neutralization of S1P and an indirect neutralization of VEGF and PDGF-B by SPHINGOMAB could prevent pericyte recruitment, BV maturation and slow the development of resistance to anti-angiogenic drugs. Targeting pericytes, in the effort to extended or increase vulnerability to anti-angiogenic agents, represents an attractive long-term approach in treating patients presenting with active CNV lesions and could promote involution of vascular complexes [Erber et al. (2004), FASEB J, vol 18: 338-40].

S1P aids in the organization of actin into cortical rings and strengthens both intracellular and cell-matrix adherence [McVerry and Garcia (2005), Cell Signal, vol 17: 131-9 and McVerry and Garcia (2004), J Cell Biochem, vol 92: 1075-85]. These structural changes correlate with decreased vascular permeability [Hla (2004), Semin Cell Dev Biol, vol 15: 513-20]. It has been demonstrated that blocking the function of S1P increased vascular permeability in kidneys, the pulmonary system and tumors [LaMontagne et al. (2006), Cancer Res, vol 66: 221-31; Sanchez et al. (2003), J Biol Chem, vol 278: 47281-90 and Awad et al. (2006), Am J Physiol Renal Physiol, vol 290: F1516-24]. Little is known however, about the permeability effects of S1P in different organ systems such as the brain and eye. Conduit ECs in the brain, and likely the eye, form tighter, less permeable barriers to fluid and solute than pulmonary artery ECs [Schnitzer et al. (1994), Biochem Biophys Res Commun, vol 199: 11-19] and most likely than kidney and tumors as well. Differential barrier functions have been attributed to a significantly greater population of focal adhesion complexes [Schnitzer et al. (1994), Biochem Biophys Res Commun, vol 199: 11-19]. In light of these differences, S1P-induced alterations in ocular vascular permeability may be less influential.

VEGF and PDGF can compromise blood-retinal barrier (BRB) integrity: SPHINGOMAB's ability to neutralize S1P trans-activation of VEGF and PDGF could prove effective in mitigating macular edema associated with AMD [Sanchez et al. (2003), J Biol Chem, vol 278: 47281-90; Saishin et al. (2003), J Cell Physiol, vol 195: 241-8 and Vinores et al. (2000), Gen Pharmacol, vol 35: 233-9]. Transgenic mice overexpressing VEGF demonstrate a BRB breakdown occurring in the area of CNV similar to that seen in AMD and diabetic retinopathies [Vinores et al. (2000), Adv Exp Med Biol, vol 476: 129-38]. Inhibitors of PDGF receptor kinase decreased leakage caused by prostaglandin-induced breakdown of the BRB [Lindahl et al. (1997), Science, vol 277: 242-5]. Finally, SPHINGOMAB mitigates the effects of bFGF and VEGF in vivo as assayed in a murine Matrigel plug model as described in the examples of this application.

S1P and fibroblast proliferation and protection from cell death: Fibroblasts respond to S1P treatment by an increase in DNA synthesis; fibroblasts transfected with Sphingosine Kinase 1 (sphK1) exhibit increased cellular proliferation [Hammer et al. (2004), J Cell Biochem, vol 91: 840-51]. Similar to the effects of S1P on several other fibroblast types (Swiss 3T3, lung and cardiac), S1P may stimulate ocular fibroblast proliferation (and subsequent differentiation). Fibroblasts are directly protected from apoptosis by addition of S1P, and apoptosis is enhanced by inhibitors of sphK1 [Olivera et al. (1999), J Cell Biol, vol 147: 545-58]. S1P blocks cytochrome C release and subsequent caspase activation [Olivera et al. (1999), J Cell Biol, vol 147: 545-58 and Kang et al. (2004), Cell Death Differ, vol 11: 1287-98]. It is established that sphK1 upregulates Akt, thereby regulating Bcl-2 family members [Limaye et al. (2005), Blood, vol 105: 3169-77] and protecting fibroblasts from apoptosis.

S1P and fibroblast migration: S1P activates signaling systems including Rho, resulting in the assembly of contractile actin filaments controlled by Rho/Rac/Cdc42 system, and leading to substantial effects on cellular migration [Radeff-Huang et al. (2004), J Cell Biochem,. Vol 92: 949-66]. The activation of Rho and Rho GTPases by S1P may be responsible for the migration of ocular fibroblasts into the wound and thereby contribute to fibrosis.

S1P and fibroblast collagen expression: S1P promotes the differentiation of quiescent fibroblasts to active myofibroblasts which exhibit enhanced collagen expression during scar formation [Urata et al. (2005), Kobe J Med Sci, vol 51: 17-27]. Concurrent with the proliferation and migration of fibroblasts into the scarring zone, myofibroblasts deposit a temporary granular network consisting primarily of osteopontin and fibronectin [Sun and Weber (2000), Cardiovasc Res, vol 46: 250-6]. As remodeling proceeds, the temporary matrix is absorbed and a collagen network established [Sun and Weber (2000), Cardiovasc Res, vol 46: 250-6]. We have demonstrated that S1P promotes collagen production by myofibroblasts. TGFβ, a well-known fibrotic mediator, has been shown to up-regulate several pro-fibrotic proteins, convert fibroblasts to myofibroblasts and stimulate inflammatory protein expression possibly through the action of S1P [Squires et al. (2005), J Mol Cell Cardiol, vol 39: 699-707 and Butt, Laurent and Bishop (1995), Eur J Cell Biol, vol 68: 330-5]. Up-regulation of TIMP1, a signaling molecule implicated in TGFβ-stimulated differentiation of fibroblasts to myofibroblasts, is blocked by siRNA against sphK1 [Yamanaka et al J Biol Chem. 2004 Dec 24;279(52):53994-4001. , suggesting that SPHINGOMAB could mitigate the profibrotic effects of TGFβ as well as mitigating the fibrogenic effects of S1P itself. Minimizing maladaptive scar formation by neutralization of S1P could be beneficial and prevent irreversible losses in visual acuity by limiting the extent of sub-retinal fibrosis and subsequent photoreceptor damage.

### F. Lysophosphatic acids (LPA)

LPA have long been known as precursors of phospholipid biosynthesis in both eukaryotic and prokaryotic cells, but LPA have emerged only recently as signaling molecules that are rapidly produced and released by activated cells, notably platelets, to influence target cells by acting on specific cell-surface receptor (see, eg, Moolenaar et al. (2004), BioEssays, vol. 26: 870-881 and van Leewen et al. (2003), Biochem Soc Trans, vol 31: 1209-1212). Besides being synthesized and processed to more complex phospholipids in the endoplasmic reticulum, LPA can be generated through the hydrolysis of pre-existing phospholipids following cell activation; for example, the sn-2 position is commonly missing a fatty acid residue due to de-acylation, leaving only the sn-3 hydroxyl esterified to a fatty acid. Moreover, a key enzyme in the production of LPA, autotaxin (lysoPLD/NPP2), may be the product of an oncogene, as many tumor types up-regulate autotaxin (Brindley (2004), J Cell Biochem, vol. 92: 900-12). The concentrations of LPA in human plasma and serum have been reported, including determinations made using sensitive and specific LC/MS procedures (Baker et al. (2001), Anal Biochem, vol 292: 287-295). For example, in freshly prepared human serum allowed to sit at 25°C for one hour, LPA concentrations have been estimated to be approximately 1.2 µM, with the LPA analogs 16:0, 18:1, 18:2, and 20:4 being the predominant species. Similarly, in freshly prepared human plasma allowed to sit at 25°C for one hour, LPA concentrations have been estimated to be approximately 0.7 µM, with 18:1 and 18:2 LPA being the predominant species.

LPA influence a wide range of biological responses, including induction of cell proliferation, stimulation of cell migration and neurite retraction, gap junction closure, and even slime mold chemotaxis (Goetzl. et al. (2002), Scientific World Journal, vol 2: 324-338). The body of knowledge about the biology of LPA continues to grow as more and more cellular systems are tested for LPA responsiveness. For instance, it is now known that, in addition to stimulating cell growth and proliferation, LPA promote cellular tension and cell-surface fibronectin binding, which are important events in wound repair and regeneration (Moolenaar et al. (2004), BioEssays, vol. 26: 870-881). Recently, anti-apoptotic activity has also been ascribed to LPA, and it has recently been reported that peroxisome proliferation receptor gamma is a receptor/target for LPA (Simon et al. (2005), J Biol Chem, vol 280: 14656-14662).

Recently, the applicants have developed several monoclonal antibodies against the LPAs. Like the anti-S1P antibody, the anti-LPA antibodies can neutralize various LPAs and mitigate their biologic and pharmacologic action. For application to ocular disease and conditions, the anti-LPA antibodies would be expected to act on the following processes for therapeutic benefit.

CNV and BV maturation: Autotaxin, a secreted lysophospholipase D responsible for producing LPAs, is essential for blood vessel formation during development [van Meeteren et al. (2006), Mol Cell Biol, vol 26: 5015-22]. In addition, unsaturated LPAs were identified as major contributors to the induction of vascular smooth muscle cell dedifferentiation [Hayashi et al. (2001), Circ Res, vol 89: 251-8].

Edema and vascular permeability: LPA induces plasma exudation and histamine release in mice [Hashimoto et al. (2006), J Pharmacol Sci, vol 100: 82-7].

Inflammation: LPA acts as inflammatory mediator in human corneal epithelial cells [Zhang et al (2006), Am J Physiol, June 7]. LPA participates in corneal wound healing [Liliom K et al (1998), Am. J. Physiol, vol 274: C1065-C1074] and stimulates the release of ROS in lens tissue [Rao et al. (2004), Molecular Visions, vol 10: 112-121]. LPA can also re-activate HSV-1 in rabbit cornea [Martin et al. (1999), Molecular Visions, vol 5: 36-42).

Fibrosis and scar formation: LPA inhibits TGFβ-mediated stimulation of type I collagen mRNA stability via an ERK-dependent pathway in dermal fibroblasts [Sato et al. (2004), Matrix Biol, vol 23: 353-61]. Moreover, LPA have some direct fibrogenic effects by stimulating collagen gene expression and proliferation of fibroblasts [Chen, et al. (2006) FEBS Lett. 580(19):4737-45.

### 3. Definitions.

Before describing the instant invention in detail, several terms used in the context of the present invention will be defined. In addition to these terms, others are defined elsewhere in the specification, as necessary. Unless otherwise expressly defined herein, terms of art used in this specification will have their art-recognized meanings.

An "immune-derived moiety" refers to any polyclonal or monoclonal antibody or antibody fragment, variant, or derivative.

An "anti-S1P antibody" or an "immune-derived moiety reactive against S1P" refers to any antibody or antibody-derived molecule that binds S1P.

An "anti-LPA antibody" or an "immune-derived moiety reactive against LPA" refers to any antibody or antibody-derived molecule that binds to all or one or more of the LPAs.

A "bioactive lipid" refers to a lipid signaling molecule. In general, a bioactive lipid does not reside in a biological membrane when it exerts its signaling effects, which is to say that while such a lipid species may exist at some point in a biological membrane (for example, a cell membrane, a membrane of a cell organelle, etc.), when associated with a biological membrane it is not a "bioactive lipid" but is instead a "structural lipid" molecule. Bioactive lipids are distinguished from structural lipids (e.g., membrane-bound phospholipids) in that they mediate extracellular and/or intracellular signaling and thus are involved in controlling the function of many types of cells by modulating differentiation, migration, proliferation, secretion, survival, and other processes. In vivo, bioactive lipids can be found in extracellular fluids, where they can be complexed with other molecules, for example serum proteins such as albumin and lipoproteins, or in "free" form, i.e., not complexed with another molecule species. As extracellular mediators, some bioactive lipids alter cell signaling by activating membrane-bound ion channels or G-protein coupled receptors that, in turn, activate complex signaling systems that result in changes in cell function or survival. As intracellular mediators, bioactive lipids can exert their actions by directly interacting with intracellular components such as enzymes and ion channels. Representative examples of bioactive lipids include LPA and S1P.

WO 2006/078336 discloses the use of a deposited inhibitory murine antibody against S1P for treating cancer, age-related macular degeneration or CNV.

Thoreson et al. (Invest. Ophthalmol. Vis. Sci., 2002, Vol. 43, 2450-2461) report that LPA is suspected to play a role in proliferative vitreoretinopathy.

Lebrun-Julien et al. (Invest. Ophthalmol. Vis. Sci., 2005, Vol. 46, E-Abstract 1319) report on the distribution of a particular LPA receptor, LPA1, in certain ocular tissues.

Anliker & Chun (JBC, 2004, Vol. 279(20), 20555-558) reviews lysophospholipid G-protein coupled receptors that bind to members of the LPA and S1P ligand family and are suspected to play a role in cell migration, CNS formation, wound healing and/or artherosclerosis and are found in various cancer cell lines, vascular endothelial cells and in the heart.

WO 02/17899 discloses antibodies directed against EDG-1, which is a receptor for S1P, and its use for the inhibition of angiogenesis.

Pyne & Pyne (Biochem. J., 2002, Vol. 349, 385-402) reviews the role of S1P in diseases and mentions cancer, angiogenesis, allergy and inflammation. Various inhibitory entities are mentioned.

The term "therapeutic agent" means an agent to mitigate angiogenesis and/or neovascularization, e.g., CNV and BV maturation; edema, vascular permeability and fibrosis, fibrogenesis and scarring associated with, or part of the underlying pathology of, ocular diseases and conditions.

The term "combination therapy" refers to a therapeutic regimen that involves the provision of at least two distinct therapies to achieve an indicated therapeutic effect. For example, a combination therapy may involve the administration of two or more chemically distinct active ingredients, for example, an anti-LPA antibody and an anti-S1P antibody. Alternatively, a combination therapy may involve the administration of an immune-derived moiety reactive against a bioactive lipid and the administration of one or more other chemotherapeutic agents. Combination therapy may, alternatively, involve administration of an anti-lipid antibody together with the delivery of another treatment, such as radiation therapy and/or surgery. Further, a combination therapy may involve administration of an anti-lipid antibody together with one or more other biological agents (e.g.,anti-VEGF, TGFβ, PDGF, or bFGF agent), chemotherapeutic agents and another treatment such as radiation and/or surgery. In the context of combination therapy using two or more chemically distinct active ingredients, it is understood that the active ingredients may be administered as part of the same composition or as different compositions. When administered as separate compositions, the compositions comprising the different active ingredients may be administered at the same or different times, by the same or different routes, using the same of different dosing regimens, all as the particular context requires and as determined by the attending physician. Similarly, when one or more anti-lipid antibody species, for example, an anti-LPA antibody, alone or in conjunction with one or more chemotherapeutic agents are combined with, for example, radiation and/or surgery, the drug(s) may be delivered before or after surgery or radiation treatment.

"Monotherapy" refers to a treatment regimen based on the delivery of one therapeutically effective compound, whether administered as a single dose or several doses over time.

The term "pharmaceutically acceptable salt" refers to salts which retain the biological effectiveness and properties of the agents and compounds of this invention and which are not biologically or otherwise undesirable. In many cases, the agents and compounds of this invention are capable of forming acid and/or base salts by virtue of the presence of charged groups, for example, charged amino and/or carboxyl groups or groups similar thereto. Pharmaceutically acceptable acid addition salts may be prepared from inorganic and organic acids, while pharmaceutically acceptable base addition salts can be prepared from inorganic and organic bases. For a review of pharmaceutically acceptable salts (see Berge, et al. (1977) J. Pharm. Sci., vol. 66, 1-19).

The terms "separated," "purified," "isolated," and the like mean that one or more components of a sample contained in a sample-holding vessel are or have been physically removed from, or diluted in the presence of, one or more other sample components present in the vessel. Sample components that may be removed or diluted during a separating or purifying step include, chemical reaction products, unreacted chemicals, proteins, carbohydrates, lipids, and unbound molecules.

The term "species" is used herein in various contexts, e.g., a particular species of chemotherapeutic agent. In each context, the term refers to a population of molecules, chemically indistinguishable from each other, of the sort referred in the particular context.

"Specifically associate" and "specific association" and the like refer to a specific, non-random interaction between two molecules, which interaction depends on the presence of structural, hydrophobic/hydrophilic, and/or electrostatic features that allow appropriate chemical or molecular interactions between the molecules.

Herein, "stable" refers to an interaction between two molecules (eg, binding of an anti-LPA or anti-S1P antibody to its target bioactive lipid) that is sufficiently strong such that the molecules can be maintained for the desired purpose or manipulation.

A "subject" or "patient" refers to an animal in which treatment can be effected by molecules of the invention. The animal may have, be at risk for, or be believed to have or be at risk for a disease or condition that can be treated by compositions and/or methods of the present invention. Animals that can be treated in accordance with the invention include vertebrates, with mammals such as bovine, canine, equine, feline, ovine, porcine, and primate (including humans and non-human primates) animals being particularly preferred examples.

A "therapeutically effective amount" (or "effective amount") refers to an amount of an active ingredient, e.g., an agent according to the invention, sufficient to effect treatment when administered to a subject or patient. Accordingly, what constitutes a therapeutically effective amount of a composition according to the invention may be readily determined by one of ordinary skill in the art. In the context of ocular therapy, a "therapeutically effective amount" is one that produces an objectively measured change in one or more parameters associated with treatment of the ocular disease or condition including an increase or decrease in the expression of one or more genes correlated with the ocular disease or condition, induction of apoptosis or other cell death pathways, clinical improvement in symptoms, a decrease in aberrant neovascularization or in inflammation, etc. Of course, the therapeutically effective amount will vary depending upon the particular subject and condition being treated, the weight and age of the subject, the severity of the disease condition, the particular compound chosen, the dosing regimen to be followed, timing of administration, the manner of administration and the like, all of which can readily be determined by one of ordinary skill in the art. It will be appreciated that in the context of combination therapy, what constitutes a therapeutically effective amount of a particular active ingredient may differ from what constitutes a therapeutically effective amount of the active ingredient when administered as a monotherapy (ie., a therapeutic regimen that employs only one chemical entity as the active ingredient).

The term "treatment" or "treating" of a disease or disorder includes preventing or protecting against the disease or disorder (that is, causing the clinical symptoms not to develop); inhibiting the disease or disorder (*i.e*., arresting or suppressing the development of clinical symptoms; and/or relieving the disease or disorder (*i.e.*, causing the regression of clinical symptoms). As will be appreciated, it is not always possible to distinguish between "preventing" and "suppressing" a disease or disorder since the ultimate inductive event or events may be unknown or latent. Accordingly, the term "prophylaxis" will be understood to constitute a type of "treatment" that encompasses both "preventing" and "suppressing." The term "treatment" thus includes "prophylaxis".

The term "therapeutic regimen" means any treatment of a disease or disorder using chemotherapeutic drugs, radiation therapy, surgery, gene therapy, DNA vaccines and therapy, antisense-based therapies including siRNA therapy, anti-angiogenic therapy, immunotherapy, bone marrow transplants, aptamers and other biologics such as antibodies and antibody variants, receptor decoys and other protein-based therapeutics.

### SUMMARY OF THE INVENTION

The invention relates to a humanized monoclonal antibody, or a fragment, variant, or derivative thereof that binds and neutralizes a bioactive lipid or bioactive lipid precursor or metabolite, wherein the bioactive lipid, bioactive lipid precursor or metabolite is sphingosine-1-phosphate (S1P) or an S1P precursor or metabolite, for use in a method for decreasing the effective concentration of said bioactive lipid in an eye of an animal in order to (i) treat or prevent aberrant fibrogenesis, fibrosis, or scarring in the eye,
(ii) modulate surgical or traumatic wound healing responses in the eye,
(iii) decrease or prevent inflammation in the eye,
(iv) decrease or prevent aberrant neovascularization of the eye,
(v) attenuate an ocular immune response in the eye, or
(vi) decrease the effective ocular concentration or activity of the bioactive lipid, bioactive lipid precursor or metabolite in order to treat or prevent an ocular disease or condition that is characterized, at least in part, by aberrant fibrogenesis, fibrosis, scarring, inflammation, aberrant neovascularization, or an immune response in the eye.

Also disclosed are methods for treating ocular diseases or conditions through administration of a pharmaceutical composition comprising an immune-derived moiety (e.g, an antibody) reactive against a bioactive lipid, in order to decrease the effective concentration so that the bioactive lipid is inhibited in whole or in part from eliciting its undesired effects. In some embodiments, the immune-derived moiety is a monoclonal antibody or fragment, variant or derivative thereof. In some embodiments, the immune-derived moiety is reactive against a lysolipid, such as S1P or LPA. Methods are also provided for decreasing or preventing aberrant fibrogenesis, fibrosis or scarring; inflammation; or aberrant neovascularization; modulating surgical and traumatic wound healing responses of the eye; or for attenuating an ocular immune response. Further provided are methods for decreasing the effective ocular concentration or activity of bioactive lipid. Also provided are methods of treating scleroderma using an immune-derived moiety reactive against a bioactive lipid, such as the lysolipids S1P or LPA. Representative bioactive lipids include sphingolipids and variants thereof such as sphingosine-1-phosphate (S1P), sphingosine, sphingosylphosphorylcholine, dihydrosphingosine. Other bioactive lysolipids include lysophosphatidic acids (LPAs) and variants thereof.

Another aspect of the present disclosure concerns pharmaceutical or veterinary compositions, including those for ocular administration, that comprise a carrier and an isolated immune-derived moiety, for example, a monoclonal antibody or antibody fragment, variant, or derivative, reactive against a bioactive lipid. Preferred carriers include those that are pharmaceutically acceptable, particularly when the composition is intended for therapeutic use in humans. For non-human therapeutic applications (e.g., in the treatment of companion animals, livestock, fish, or poultry), veterinarily acceptable carriers may be employed.

Exemplary routes of administration of an immune-derived moiety according to the invention, preferably as part of a therapeutic composition, include systemic administration, parenteral administration (e.g., via injection via an intravenous, intramuscular, or subcutaneous route), transdermal, intradermal or transmucosal delivery, intraocular or periocular injection, mucosal or topical administration or by inhalation.

These and other aspects and embodiments are discussed in greater detail in the sections that follow.

### BRIEF DESCRIPTION OF THE DRAWINGS

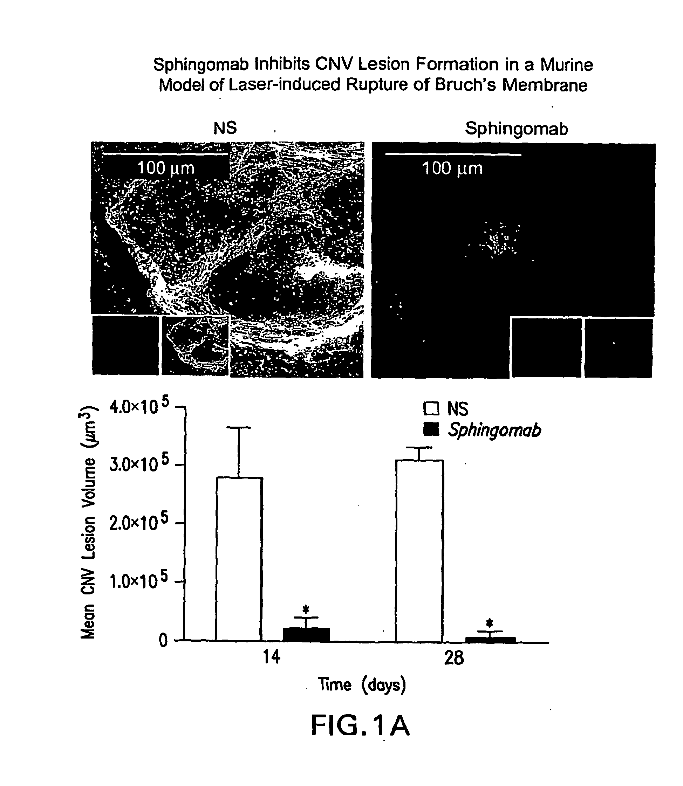

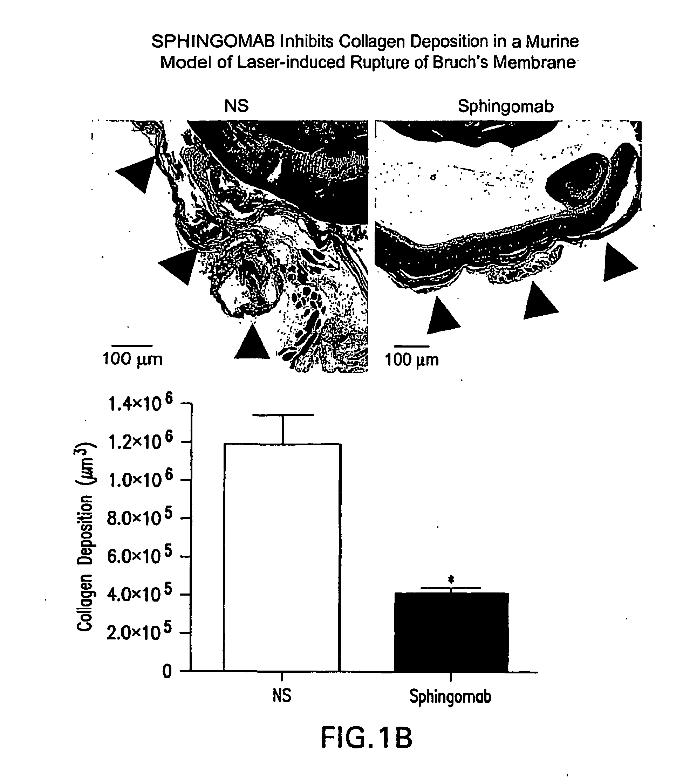

Figure 1: SPHINGOMAB reduced CNV and scar formation in ocular lesions. Mice were treated with SPHINGOMAB or an isotype-matched non-specific mAb. CNV lesions were induced by laser rupture of Bruchs membrane. Shown are graphs and representative images of lesions from each treatment group stained with rhodamine-conjugated R. communis agglutinin I for vascularization (A) or Masson's Trichrome for collagen scar formation (B). **Figure 1a** shows that SPHINGOMAB dramatically attenuates choroidal neovascularization 14 and 28 days after laser-induced rupture of Bruch's membrane.**Figure 1b** shows that SPHINGOMAB significantly reduces fibrosis associated with CNV lesion formation 28 days after laser-induced rupture of Bruchs's membrane.

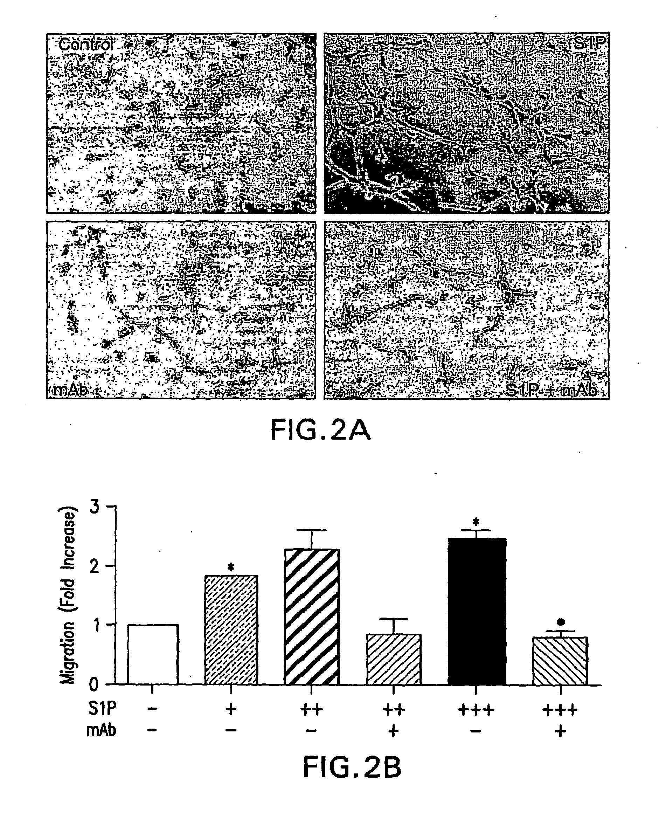

**Figure 2****:** S1P promotes neovascularization through induction of HUVECs tube formation and migration and is reduced by SPHINGOMAB. Panel A: Micrographs of HUVECs seeded on Matrigel and incubated for 6 hrs to evaluate tube formation. Panel B: HUVECs were treated with 1µM S1P ± SPHINGOMAB (1µg/ml) for 6 hrs in a Matrigel invasion chamber. The number of cells that migrated to the Matrigel membrane were counted in 5 independent fields.

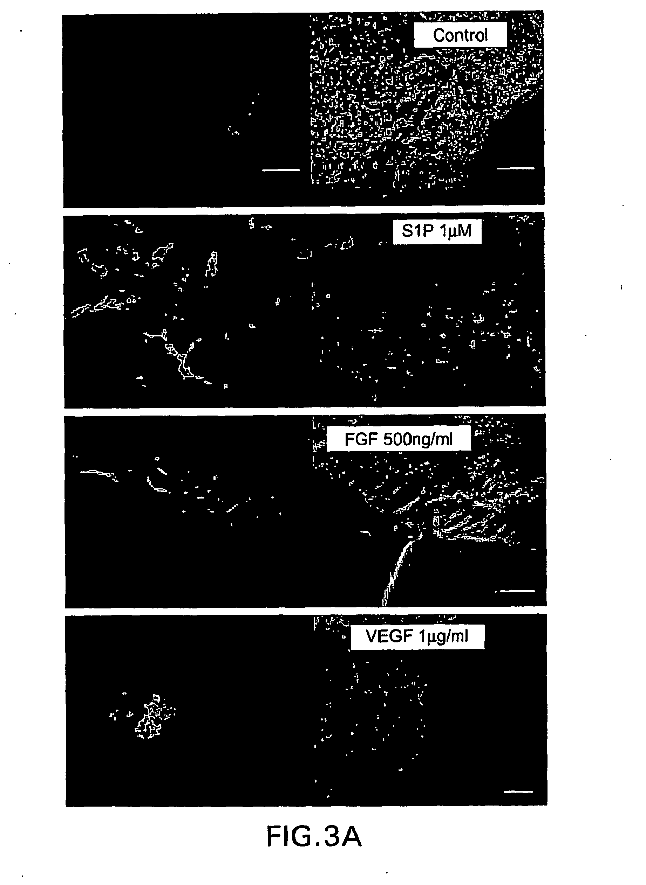

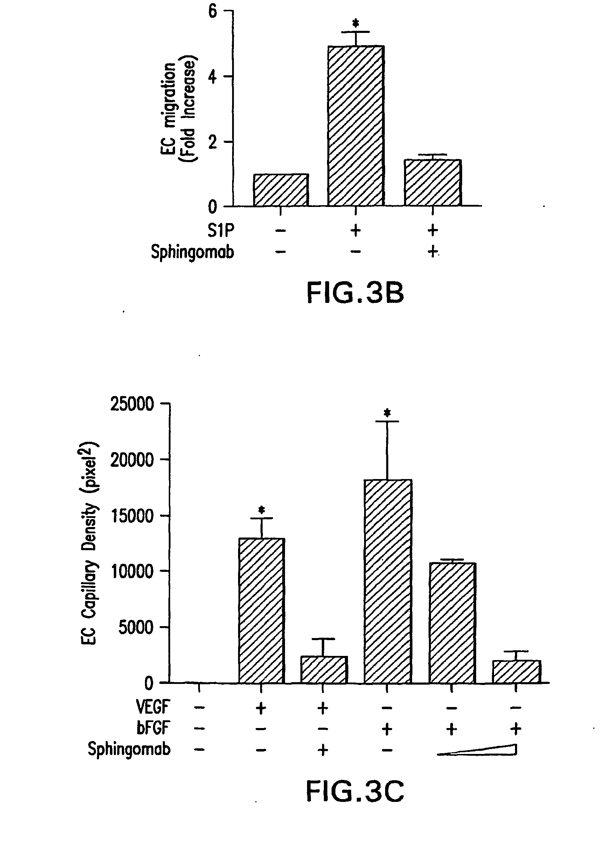

**Figure 3****.** SPHINGOMAB neutralizes S1P-, VEGF- and bFGF-induced neovascularization. A: Representative FITC-stained BVs from sections of Matrigel plugs ±GFs. B: S1P stimulates EC infiltration. C: Quantification of relative fluorescence from Matrigel plugs stimulated with VEGF or bFGF as an indicator of neovascularization. S1P, VEGF and bFGF's effects were inhibited when mice were systemically treated with 1 or 25mg/kg of SPHINGOMAB.

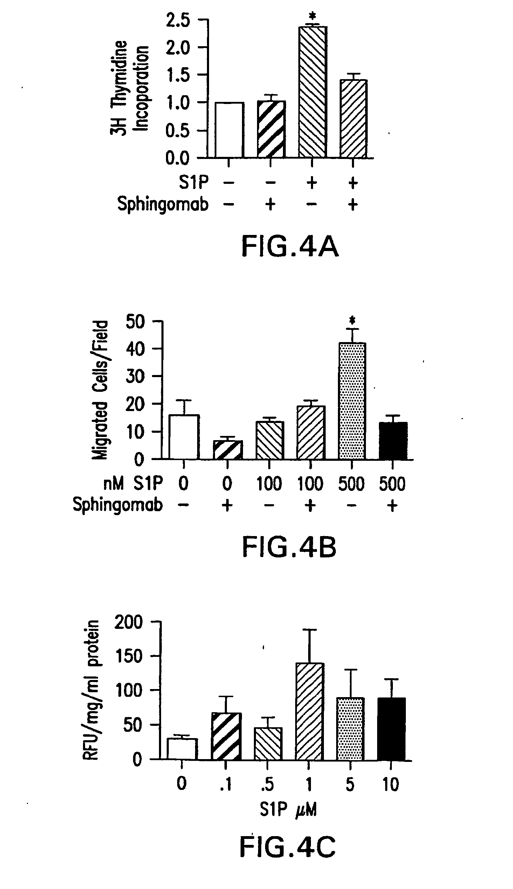

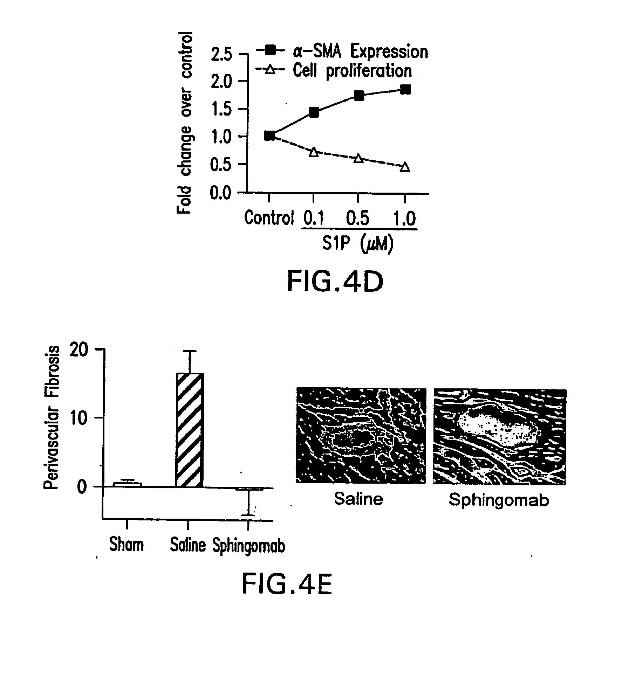

**Figure 4****.** SPHINGOMAB neutralized S1P-stimulated scar formation. Fibroblasts were serum-starved and then treated with 0, 0.1, 0.5 or 1µM S1P +/- 1µg/mL SPHINGOMAB for 12-24 hrs. S1P stimulated Swiss 3T3 fibroblast proliferation as measured by 3H-thymidine incorporation (A), murine cardiac fibroblast migration in a scratch assay (B), collagen gene expression (relative fluorescence) in isolated cardiac fibroblasts from transgenic mice expressing collagen-GFP (C) and WI-38 cell differentiation into myofibroblasts as measured by decreased cellular proliferation and increased α-SMA expression (D); SPHINGOMAB neutralized each of S1P's effects. SPHINGOMAB reduced perivascular fibrosis in vivo in a murine model of a permanent myocardial infarction (E).

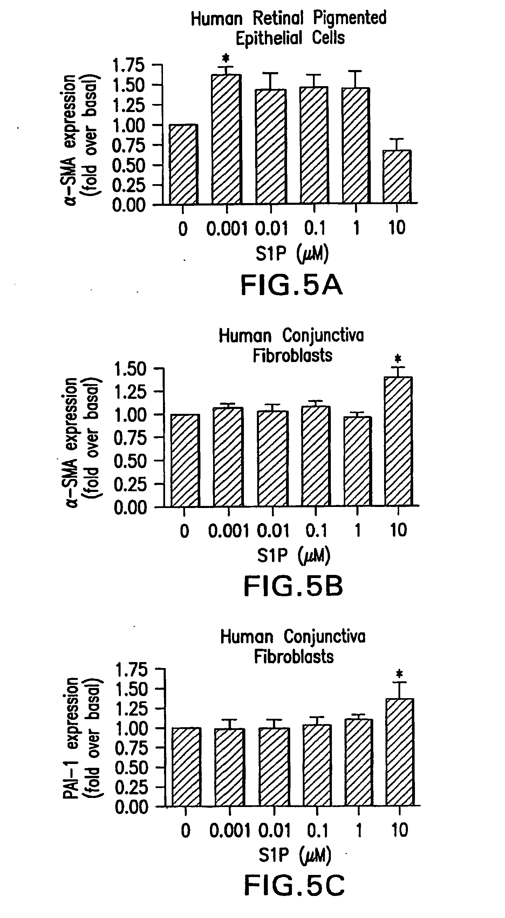

**Figure 5****.** S1P promotes transformation of ocular epithelial cells and fibroblasts into contractile, scar tissue-producing myofibroblasts. The effects of S1P on myofibroblast transformation of several human ocular cell lines were examined. S1P was found to stimulate production of α-Smooth muscle actin (α-SMA; a myofibroblast marker) in human retinal pigmented epithelial cells (Figure 5A) and human conjunctiva fibroblasts (Figure 5B). These data demonstrate for the first time, that S1P is among the factors that promote transformation of ocular epithelial cells and fibroblasts into contractile, scar tissue-producing myofibroblasts. The effects of S1P on expression of plasminogen activator inhibitor (PAI-1) in human conjunctiva fibroblasts were also examined. Increased PAI-1 expression correlates with a decrease in the proteolytic degradation of connective tissue and is upregulated in association with several fibrotic diseases that involve increased scarring. As shown in Figure 5C, S1P stimulates the PAI-1 expression in a dose-dependent manner.

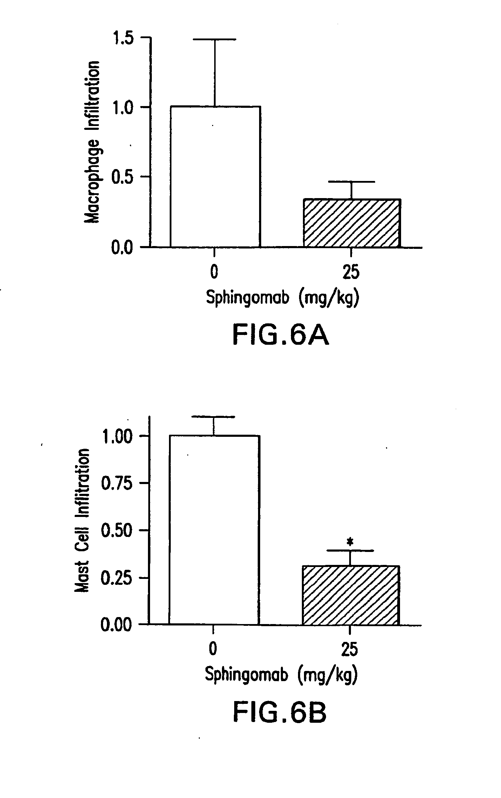

**Figure 6****.** SPHINGOMAB reduced immune-cell wound infiltration *in vivo*. Mice were subjected to MI, treated with saline or 25mg/kg SPHINGOMAB 48 hrs after surgery and then sacrificed on day 4. SPHINGOMAB reduced macrophage (A) and mast cell (B) infiltration into the wound. Data are represented as fold decrease of saline treated values.

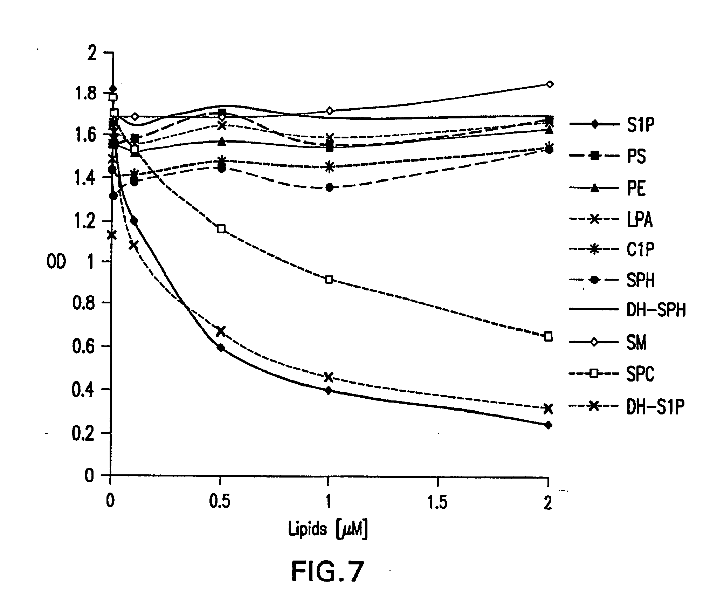

**Figure 7****.** SPHINGOMAB is highly specific for S1P. A graph based on competitive ELISA demonstrates SPHINGOMAB's specificity for S1P compared to other bioactive lipids. SPHINGOMAB demonstrated no cross-reactivity to sphingosine (SPH), the immediate metabolic precursor of S1P or lysophosphatidic acid (LPA), an important extracellular signaling molecule that is structurally and functionally similar to S1P. SPHINGOMAB did not recognize other structurally similar lipids and metabolites, including ceramide-1-phosphate (C1P), dihydrosphingosine (DH-SPH), phosphatidyl serine (PS), phosphatidyl ethanolamine (PE), or sphingomyelin (SM). SPHINGOMAB did cross react with dihydrosphingosine-1-phosphate (DH-S1P) and, to a lesser extent, sphingosylphoryl choline (SPC). The affinity (Kd) of SPHINGOMAB for S1P is <100pM, much higher than most therapeutic antibodies, particularly other molecular sponges.

### DETAILED DESCRIPTION OF THE INVENTION

### 1. Compounds

The term "immune-derived moiety," which includes antibodies (Ab) or immunoglobulins (Ig), refers to any form of a peptide, polypeptide derived from, modeled after or encoded by, an immunoglobulin gene, or a fragment of such peptide or polypeptide that is capable of binding an antigen or epitope [see, eg, Immunobiology, 5th Edition, Janeway, Travers, Walport, Shlomchiked. (editors), Garland Publishing (2001)]. In the present invention, the antigen is a bioactive lipid molecule. Antibody molecules or immunoglobulins are large glycoprotein molecules with a molecular weight of approximately 150 kDa, usually composed of two different kinds of polypeptide chain. One polypeptide chain, termed the "heavy" chain (H) is approximately 50 kDa. The other polypeptide, termed the "light" chain (L), is approximately 25 kDa. Each immunoglobulin molecule usually consists of two heavy chains and two light chains. The two heavy chains are linked to each other by disulfide bonds, the number of which varies between the heavy chains of different immunoglobulin isotypes. Each light chain is linked to a heavy chain by one covalent disulfide bond. In any given naturally occurring antibody molecule, the two heavy chains and the two light chains are identical, harboring two identical antigen-binding sites, and are thus said to be divalent, i.e., having the capacity to bind simultaneously to two identical molecules.

The "light" chains of antibody molecules from any vertebrate species can be assigned to one of two clearly distinct types, kappa (k) and lambda (1), based on the amino acid sequences of their constant domains. The ratio of the two types of light chain varies from species to species. As a way of example, the average k to 1 ratio is 20:1 in mice, whereas in humans it is 2:1 and in cattle it is 1:20.

The "heavy" chains of antibody molecules from any vertebrate species can be assigned to one of five clearly distinct types, called isotypes, based on the amino acid sequences of their constant domains. Some isotypes have several subtypes. The five major classes of immunoglobulin are immunoglobulin M (IgM), immunoglobulin D (IgD), immunoglobulin G (IgG), immunoglobulin A (IgA), and immunoglobulin E (IgE). IgG is the most abundant isotype and has several subclasses (IgG1, 2, 3, and 4 in humans). The Fc fragment and hinge regions differ in antibodies of different isotypes, thus determining their functional properties. However, the overall organization of the domains is similar in all isotypes.

The term "variable region" refers to the N-terminal portion of the antibody molecule or a fragment thereof. In general, each of the four chains has a variable (V) region in its amino terminal portion, which contributes to the antigen-binding site, and a constant (C) region, which determines the isotype. The light chains are bound to the heavy chains by many noncovalent interactions and by disulfide bonds and the V regions of the heavy and light chains pair in each arm of antibody molecule to generate two identical antigen-binding sites. Some amino acid residues are believed to form an interface between the light- and heavy-chain variable domains [see Kabat et al. (1991), Sequences of Proteins of Immunological Interest, Fifth Edition, National Institute of Health, Bethesda, Md. and Clothia et al. (1985), J. Mol. Biol, vol 186: 651].

Of note, variability is not uniformly distributed throughout the variable domains of antibodies, but is concentrated in three segments called "complementarity-determining regions" (CDRs) or "hypervariable regions" both in the light-chain and the heavy-chain variable domains. The more highly conserved portions of variable domains are called the "framework region" (FR). The variable domains of native heavy and light chains each comprise four FR regions connected by three CDRs. The CDRs in each chain are held together in close proximity by the FR regions and, with the CDRs from the other chains, form the antigen-binding site of antibodies [see Kabat et al. (1991), Sequences of Proteins of Immunological Interest, Fifth Edition, National Institute of Health, Bethesda, Md.]. Collectively, the 6 CDRs contribute to the binding properties of the antibody molecule for the antigen. However, even a single variable domain (or half of an Fv, comprising only three CDRs specific for an antigen) has the ability to recognize and bind antigen [see Pluckthun (1994), in The Pharmacology of Monoclonal Antibodies, vol. 113, Rosenburg and Moore eds., Springer-Verlag, New York, pp. 269-315].

The term "constant domain" refers to the C-terminal region of an antibody heavy or light chain. Generally, the constant domains are not directly involved in the binding properties of an antibody molecule to an antigen, but exhibit various effector functions, such as participation of the antibody in antibody-dependent cellular toxicity. Here, "effector functions" refer to the different physiological effects of antibodies (e.g., opsonization, cell lysis, mast cell, basophil and eosinophil degranulation, and other processes) mediated by the recruitment of immune cells by the molecular interaction between the Fc domain and proteins of the immune system. The isotype of the heavy chain determines the functional properties of the antibody. Their distinctive functional properties are conferred by the carboxy-terminal portions of the heavy chains, where they are not associated with light chains.

As used herein, "antibody fragment" refers to a portion of an intact antibody that includes the antigen binding site or variable regions of an intact antibody, wherein the portion can be free of the constant heavy chain domains (e.g., CH2, CH3, and CH4) of the Fc region of the intact antibody. Alternatively, portions of the constant heavy chain domains (e.g., CH2, CH3, and CH4) can be included in the "antibody fragment". Examples of antibody fragments are those that retain antigen-binding and include Fab, Fab', F(ab')2, Fd, and Fv fragments; diabodies; triabodies; single-chain antibody molecules (sc-Fv); minibodies, nanobodies, and multispecific antibodies formed from antibody fragments. By way of example, a Fab fragment also contains the constant domain of a light chain and the first constant domain (CH1) of a heavy chain.

The term "variant" refers to an amino acid sequence which differs from the native amino acid sequence of an antibody by at least one amino acid residue or modification. A native or parent or wild-type amino acid sequence refers to the amino acid sequence of an antibody found in nature. "Variant" of the antibody molecule includes, but is not limited to, changes within a variable region or a constant region of a light chain and/or a heavy chain, including the hypervariable or CDR region, the Fc region, the Fab region, the CH₁ domain, the CH₂ domain, the CH₃ domain, and the hinge region.

The term "specific" refers to the selective binding of an antibody to its target epitope. Antibody molecules can be tested for specificity of binding by comparing binding of the antibody to the desired antigen to binding of the antibody to unrelated antigen or analogue antigen or antigen mixture under a given set of conditions. Preferably, an antibody according to the invention will lack significant binding to unrelated antigens, or even analogs of the target antigen. Here, the term "antigen" refers to a molecule that is recognized and bound by an antibody molecule or immune-derived moiety that binds to the antigen. The specific portion of an antigen that is bound by an antibody is termed the "epitope." A "happen" refers to a small molecule that can, under most circumstances, elicit an immune response (i.e., act as an antigen) only when attached to a carrier molecule, for example, a protein, polyethylene glycol (PEG), colloidal gold, silicone beads, and the like. The carrier may be one that also does not elicit an immune response by itself.

The term "antibody" is used in the broadest sense, and encompasses monoclonal, polyclonal, multispecific (e.g., bispecific, wherein each arm of the antibody is reactive with a different epitope or the same or different antigen), minibody, heteroconjugate, diabody, triabody, chimeric, and synthetic antibodies, as well as antibody fragments that specifically bind an antigen with a desired binding property and/or biological activity.

The term "monoclonal antibody" (mAb) refers to an antibody, or population of like antibodies, obtained from a population of substantially homogeneous antibodies, and is not to be construed as requiring production of the antibody by any particular method. For example, monoclonal antibodies can be made by the hybridoma method first described by Kohler and Milstein (1975), Nature, vol 256: 495-497, or by recombinant DNA methods.

The term "chimeric" antibody (or immunoglobulin) refers to a molecule comprising a heavy and/or light chain which is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity [Cabilly *et al.* (1984), infra; Morrison et al., Proc. Natl. Acad. Sci. U.S.A. 81:6851].

The term "humanized antibody" refers to forms of antibodies that contain sequences from non-human (eg, murine) antibodies as well as human antibodies. A humanized antibody can include conservative amino acid substitutions or non-natural residues from the same or different species that do not significantly alter its binding and/or biologic activity. Such antibodies are chimeric antibodies that contain minimal sequence derived from non-human immunoglobulins. For the most part, humanized antibodies are human immunoglobulins (recipient antibody) in which residues from a complementary-determining region (CDR) of the recipient are replaced by residues from a CDR of a non-human species (donor antibody) such as mouse, rat, camel, bovine, goat, or rabbit having the desired properties. Furthermore, humanized antibodies can comprise residues that are found neither in the recipient antibody nor in the imported CDR or framework sequences. These modifications are made to further refine and maximize antibody performance. Thus, in general, a humanized antibody will comprise all of at least one, and in one aspect two, variable domains, in which all or all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), or that of a human immunoglobulin. See, e.g., Cabilly et al., U.S. Pat. No. 4,816,567; Cabilly *et al*., European Patent No. 0,125,023 B1; Boss et al., U.S. Pat. No. 4,816,397; Boss *et al*., European Patent No. 0,120,694 B1; Neuberger, M. S. et al., WO 86/01533; Neuberger, M. S. *et al.,* European Patent No. 0,194,276 B1; Winter; U.S. Pat. No. 5,225,539; Winter, European Patent No. 0,239,400 B1; Padlan, E. A. *et al.,* European Patent Application No. 0,519,596 A1; Queen et al. (1989) Proc. Nat'l Acad. Sci. USA, vol 86:10029-10033).

The term "bispecific antibody" can refer to an antibody, or a monoclonal antibody, having binding properties for at least two different epitopes. In one embodiment, the epitopes are from the same antigen. In another embodiment, the epitopes are from two different antigens. Methods for making bispecific antibodies are known in the art. For example, bispecific antibodies can be produced recombinantly using the co-expression of two immunoglobulin heavy chain/light chain pairs. Alternatively, bispecific antibodies can be prepared using chemical linkage. Bispecific antibodies include bispecific antibody fragments.

The term "heteroconjugate antibody" can refer to two covalently joined antibodies. Such antibodies can be prepared using known methods in synthetic protein chemistry, including using crosslinking agents. As used herein, the term "conjugate" refers to molecules formed by the covalent attachment of one or more antibody fragment(s) or binding moieties to one or more polymer molecule(s).

The term "biologically active" refers to an antibody or antibody fragment that is capable of binding the desired epitope and in some way exerting a biologic effect. Biological effects include, but are not limited to, the modulation of a growth signal, the modulation of an anti-apoptotic signal, the modulation of an apoptotic signal, the modulation of the effector function cascade, and modulation of other ligand interactions.

The term "recombinant DNA" refers to nucleic acids and gene products expressed therefrom that have been engineered, created, or modified by man. "Recombinant" polypeptides or proteins are polypeptides or proteins produced by recombinant DNA techniques, for example, from cells transformed by an exogenous DNA construct encoding the desired polypeptide or protein. "Synthetic" polypeptides or proteins are those prepared by chemical synthesis.

The term "expression cassette" refers to a nucleotide molecule capable of affecting expression of a structural gene (i.e., a protein coding sequence, such as an antibody of the invention) in a host compatible with such sequences. Expression cassettes include at least a promoter operably linked with the polypeptide-coding sequence, and, optionally, with other sequences, e.g., transcription termination signals. Additional regulatory elements necessary or helpful in effecting expression may also be used, e.g., enhancers. Thus, expression cassettes include plasmids, expression vectors, recombinant viruses, any form of recombinant "naked DNA" vector, and the like.

### 2. Applications

Disclossed herein are compositions and methods for treating or preventing ocular diseases and conditions, using one or more therapeutic agents that alter the activity or concentration of one or more undesired bioactive lipids, or precursors or metabolites thereof. The therapeutic methods and compositions act by changing the effective concentration, i.e., the absolute, relative, effective and/or available concentration and/or activities, of certain undesired bioactive lipids. Lowering the effective concentration of the bioactive lipid may be said to "neutralize" the target lipid or its undesired effects, including downstream effects. Here, "undesired" refers to a bioactive lipid that is unwanted due to its involvement in a disease process, for example, as a signaling molecule, or to an unwanted amount of a bioactive lipid which contributes to disease when present in excess.

Without wishing to be bound by any particular theory, it is believed that inappropriate concentrations of lipids such as S1P and /or LPA, and/or their metabolites or downstream effectors, may cause or contribute to the development of various ocular diseases and disorders. As such, the compositions and methods can be used to treat these ocular diseases and disorders, particularly by decreasing the effective in vivo concentration of a particular target lipid, for example, S1P and /or LPA. In particular, it is believed that the compositions and methods of the invention are useful in treating ocular diseases characterized, at least in part, by aberrant neovascularization, angiogenesis, fibrogenesis, fibrosis, scarring, inflammation, and immune response.

Examples of several classes of ocular diseases that may be treated in accordance with the invention are described below. It will be appreciated that many disease and conditions are characterized, at least in part, by multiple pathological processes (for example, both pathological neovascularization and scarring) and that the classifications provided herein are for descriptive convenience and do not limit the invention.

### Ischemic Retinopathies associated with pathologic neovascularization and diseases characterized by epiretinal and or subretinal membrane formation

Ischemic retinopathies (IR) are a diverse group of disorders characterized by a compromised retinal blood flow. Examples of IR include diabetic retinopathy (DR), retinopathy of prematurity (ROP), sickle cell retinopathy and retinal venous occlusive disease. All of these disorders can be associated with a VEGF driven proliferation of pathological retinal neovascularization which can ultimately lead to intraocular hemorrhaging, epi-retinal membrane formation and tractional retinal detachment. Idiopathic epi-retinal membranes (ERMs), also called macular pucker or cellophane retinopathy, can cause a reduction in vision secondary to distortion of the retinal architecture. These membranes sometimes recur despite surgical removal and are sometimes associated with retinal ischemia. VEGF and its receptors are localized to ERMs. The presence of VEGF in membranes associated with proliferative diabetic retinopathy, proliferative vitreoretinopathy and macular pucker further suggests that this cytokine plays an important role in angiogenesis in ischemic retinal disease and in membrane growth in proliferative vitreoretinal disorders. In addition VEGF receptors, VEGFR1 and VEGFR2 are also identified on cells in ERMs. These data show that VEGF may be an autocrine and/or paracrine stimulator that may contribute to the progression of vascular and avascular ERMs. PDGF and its receptors [Robbins et al. (1994), Invest Ophthalmol Vis Sci; vol 35: 3649-3663] has been described in eyes with proliferative retinal diseases [Cassidy et al. (1998), Br J Ophthamol; vol 82: 181-85 and Freyberger et al. (2000), Exp Clin Endocrinol Diabetes, vol 108: 106-109]. These findings suggest that PDGF ligands and receptors are widespread in proliferative retinal membranes of different origin and suggest that autocrine and paracrine stimulation with PDGF may be involved in ERM pathogenesis. Transforming growth factor-β (TGF-β) is involved in the formation of ERMs [Poumaras et al. (1998), Klin Monatsbl Augenheilkd, vol 212: 356-358] as demonstrated by TGF staining and immunoreactivity. In addition, TGF-β receptor II is expressed in myofibroblasts of ERM of diabetic and PVR membranes. These results suggest that TGF-β, produced in multiple cell types in retina and ERMs, is an attractive target for the treatment of PVR, diabetic and secondary ERMs. Interleukin-6 (IL-6) has been reported to be increased in human vitreous in proliferative diabetic retinopathy (PDR) [La Heij et al. (2002), Am J Ophthal, 134: 367-375] and in one study 100% of the diabetic ERMs studied expressed IL-6 protein [Yamamoto et al. (2001) Am J Ophthal, vol 132: 369-377].

Exogenous administration of basic fibroblastic growth factor (bFGF) has been shown to induce endothelial proliferation and VEGF expression [Stavri et al. (1995), Circulation, vol 92: 11-14]. Consistent with these observations, bFGF concentration is increased in vitreous samples from patients with PDR [Sivalingam et al. (1990), Arch Ophthalmol, vol 108: 869-872 and Boulton et al. (1997), Br J Ophthalmol, vol 81: 228-233]. bFGF is also involved in the formation of ERMs [Hueber et al. (1996), Int. Ophthalmol, vol 20: 345-350] demonstrated bFGF in 8 out of 10 PDR membranes studied. Moreover, these workers found positive staining for the corresponding receptor, FGFR1. Immunoreactivity for bFGF has also been demonstrated in nonvascular idiopathic ERMs. These results implicate bFGF in the formation of both vascular and avascular ERMs. [Harada et al. (2006), Prog in Retinal and Eye Res, vol 25; 149-164]. Elevated bFGF has also been detected in the serum of patients with ROP (Becerril et al. (2005), Ophthalmology, vol 112, 2238].

Given the known pleotropic effects of S1P and its interactions with VEGF, bFGF, PDGF, TGF-β and IL-6, it is believed that an agent that binds, antagonizes, inhibits the effects or the production of S1P will be effective at suppressing pathologic retinal neovascularization in ischemic retinopathies and posterior segment diseases characterized by vascular or avascular ERM formation. Other ocular conditions characterized, at least in part, by aberrant neovascularization or angiogenesis include age-related macular degeneration, corneal graft rejection, neovascular glaucoma, contact lens overwear, infections of the cornea, including herpes simplex, herpes zoster and protozoan infection, pterygium, infectious uveitis, chronic retinal detachment, laser injury, sickle cell retinopathy, venous occlusive disease, choroidal neovascularization, retinal angiomatous proliferation, and idiopathic polypoidal choroidal vasculopathy.

### Proliferative vitreoretinopathy (PVR)

PVR is observed after spontaneous rhegmatogenous retinal detachment and after traumatic retinal detachment. It is a major cause of failed retinal detachment surgery. It is characterized by the growth and contraction of cellular membranes on both sides of the retina, on the posterior vitreous surface and the vitreous base. This excessive scar tissue development in the eye may lead to the development of tractional retinal detachment, and therefore treatments directed at the prevention or inhibition of proliferative vitreoretinopathy (PVR) are a logical principle of management of retinal detachment. Histopathologically PVR is characterized by excessive collagen production, contraction and cellular proliferation [Michels, Retinal Detachment 2nd Edition. Wilkinsin CP, Rice TA Eds, Complicated types of retinal detachment, pp 641-771, Mosby St Louis 1997]. Cellular types identified in PVR membranes include mainly retinal pigmented epithelial cells, fibroblasts, macrophages and vascular endothelial cells [Jerdan JA et al. (1989), Ophthalmology, vol 96: 801-10 and Vidinova et al. (2005), Klin Monatsbl Augenheilkd; vol 222:568-571]. The pathophysiology of this excessive scarring reaction appears to be mediated by a number of cytokines including platelet derived growth factor (PDGF), transforming growth factor (TGF) beta, basic fibroblastic growth factor (bFGF), interleukin -6 (IL)-6 and interleukin-8 (IL)-8 [Nagineni et al. (2005), J Cell Physiol, vol 203: 35-43; La Heij et al (2002), Am J Ophthalmol, 134: 367-75; Planck et al. (1992), Curr Eye Res; vol 11: 1031-9; Canataroglu et al. (2005) Ocul Immunol Inflamm; vol 13: 375-81 and Andrews et al. (1999) Ophthalmol Vis Sci; vol 40: 2683-9]. Inhibition of these cytokines may help prevent the development of PVR if given in a timely fashion or limit its severity [Akiyama et al (2006), J Cell Physiol, vol 207:407-12 and Zheng Y et al (2003), Jpn J Ophthalmolm, vol 47:158-65].

Sphingosine -1-Phosphate (S1P) is a bioactive lysolipid with pleotrophic effects. It is pro-angiogenic, pro inflammatory (stimulates the recruitment of macrophages and mast cells) and pro-fibrotic (stimulates scar formation). S1P generally stimulates cells to proliferate and migrate and is anti-apoptotic. S1P achieves these biologically diverse functions through its interactions with numerous cytokines and growth factors. Inhibition of S1P via a monoclonal antibody (SPHINGOMAB) has been demonstrated to block the functions of vascular endothelial growth factor (VEGF), bFGF, IL-6 and IL-8 [Visentin B et al. (2006), Cancer Cell, vol 9: 1-14]. Binding of S1P to the S1P₁ receptor can also increase PDGF production; therefore an agent that binds S1P would also be expected to diminish PDGF production [Milstien and Spiegel (2006), Cancer Cell, vol 9:148-150]. As shown in the Examples below, it has now been demonstrated that in vitro S1P transforms human RPE cells into a myofibroblast-like phenotype similar to the type seen in PVR. Given the pathophysiology that ultimately results in the excessive scarring seen in PVR and the known effects of S1P on these same key mediators, it is believed that an agent that binds, antagonizes, or inhibits the effects or the production of S1P will be effective at eliminating or minimizing the development of PVR and its severely damaging effects on the eye.

### Uveitis

Uveitis is an inflammatory disorder of the uveal tract of the eye. It can affect the front (anterior) or back (posterior) of the eye or both. It can be idiopathic or infectious in etiology and can be vision-threatening. Idiopathic uveitis has been associated with increased CD4+ expression in the anterior chamber. [Calder et al. (1999), Invest Ophthalmol Vis Sci, vol 40: 2019-24]. Data also suggests a pathologic role of the T lymphocyte and its chemoattractant IP-10 in the pathogenesis of uveitis [Abu El-Asrar (2004), Am J Ophthalmol, vol 138: 401-11]: Other chemokines in acute anterior uveitis include macrophage inflammatory proteins, monocyte chemoattractant protein-1 and IL-8. These cytokines probably play a critical role in leukocyte recruitment in acute anterior uveitis. [Verma et al. (1997), Curr Eye Res; vol 16; 1202-8]. Given the profound and pleiotropic effects of the S1P signaling cascade, it is believed that SPHINGOMAB and other immune moieties that reduce the effective concentration of bioactive lipid would serve as an effective method of reducing or modulating the intraocular inflammation associated with uveitis.

### Refractive surgery

The corneal wound healing response is of particular relevance for refractive surgical procedures since it is a major determinant of safety and efficacy. These procedures are performed for the treatment of myopia, hyperopia and astigmatism. Laser in situ keratomileusis (LASIK) and photorefractive keratectomy (PRK) are the most common refractive procedures however others have been developed in an attempt to overcome complications. These complications include overcorrection, undercorrection, regression and stromal opacification among others. A number of common complications are related to the healing response and have their roots in the biologic response to surgery. One of the greatest challenges in corneal biology is to promote tissue repair via regeneration rather than fibrosis. It is believed that the choice between regeneration and fibrosis lies in the control of fibroblast activation. [Stramer et al (2003), Invest Ophthalmol Vis Sci; vol 44: 4237-4246 and Fini (1999) Prog Retin Eye Res, vol 18: 529-551]. Cells called myofibroblasts may appear in the subepithelial stroma 1-2 weeks after surgery or injury. Myofibroblasts are presumably derived from keratocytes under the influence of TGF-β [Jester et al (2003) Exp Eye Res, vol 77: 581-592]. Corneal haze and stromal scarring are characterized by reduced corneal transparency and may be associated with fibroblast and myofibroblast generation. In situ and in vitro studies have suggested that TGF-β and PDGF are important in stimulating myofibroblast differentiation [Folger et al. (2001), Invest Ophthalmol Vis Sci; 42: 2534-2541]. Haze can be noted in the central interface after LASIK under certain circumstances. These include diffuse lamellar keratitis, donut-shaped flaps, and retention of epithelial debris at the interface. It is likely that each of these is associated with increased access of TGF-β from epithelial cells to the activated keratocytes. [Netto et al. (2005), Cornea, vol 24: 509-522]. Regression is most likely due to heightened epithelial-stromal wound healing interactions such as increased production of epithelium modulating growth factors by corneal fibroblasts and or myofibroblasts [Netto et al. (2005), Cornea, vol 24: 509-522]. Inhibition of TGF-β binding to receptors with topical anti-TGF-β antibody has been shown to reduce haze induced by PRK [Jester et al. (1997), Cornea, vol 16: 177-187]. Given the known effects of anti-bioactive lipid antibody on the fibrotic process and TGF-β, we believe that it may aid in treating some of the complications of refractive surgery such as haze, stromal scarring and regression.

### Modulation of Glaucoma Filtration Surgery

Glaucoma is classically thought of a disease whereby elevated intraocular pressure causes damage to the optic nerve and ultimately compromises the visual field and or the visual acuity. Other forms of glaucoma exist where optic nerve damage can occur in the setting of normal pressure or so called "normal tension glaucoma". For many patients medications are able to control their disease, but for others glaucoma filtration surgery is needed whereby a fistula is surgically created in the eye to allow fluid to drain. This can be accomplished via trabeculectomy, the implantation of a medical device or other methods of surgical intervention. Glaucoma filtration surgery fails due to a wound healing process characterized by the proliferation of fibroblasts and ultimately scarring. Anti-metabolites such as 5-fluorouracil and mitomycin C can reduce subsequent scarring; however, even with the use of these drugs long term follow up shows that surgical failure is still a serious clinical problem. [Mutsch and Grehn (2000), Graefes Arch Clin Exp Ophthalmol; vol 238: 884-91 and Fontana et al. (2006), Ophthalmology, vol 113: 930-936]. Studies of human Tenon's capsule fibroblasts demonstrate that they have the capacity to synthesize bFGF and PDGF and TGF-β and that these growth factors are implicated in the tissue repair process after glaucoma filtration surgery that contributes to the failure of the procedure. [Trpathi et al. (1996), Exp Eye Res, vol 63: 339-46]. Additional studies have also implicated these growth factors in the post filtration wound response [Denk et al. (2003), Curr Eye Res; vol 27: 35-44] concluded that different isoforms of PDGF are major stimulators of proliferation of Tenon's capsule fibroblasts after glaucoma filtration surgery while TGF-β is essential for the transformation of Tenon's capsule fibroblasts into myofibroblasts. We have demonstrated that S1P is present in human Tenon's capsule/conjunctival fibroblasts and that S1P is strongly expressed in the wound healing response. S1P also stimulates the profibrotic function of multiple fibroblast cell types and the transformation into the myofibroblast phenotype and collagen production. Given the specific pleotropic effects of S1P and its known interactions with bFGF, PDGF and TGF-beta, it is believed that an agent that binds, antagonizes, inhibits the effects or the production of S1P, or perhaps other bioactive lipids such as LPA, will be effective at modulating the wound healing and/or fibrotic response that leads to failure of glaucoma surgery and will be an effective therapeutic method of enhancing successful surgical outcomes. It is envisioned that the agent could be administered, e.g., via intravitreal or subconjunctival injection or topically.

### Corneal transplantation

Corneal transplantation (penetrating keratoplasty (PK)) is the most successful tissue transplantation procedure in humans. Yet of the 47,000 corneal transplants performed annually in the United States, corneal allograft rejection is still the leading cause of corneal graft failure. [Ing JJ et al. (1998), Ophthalmology, vol 105: 1855-1865]. Currently, we do not sufficiently have the ability to avert allograft rejection although immunosuppression and immunomodulation may be a promising approach. Recently it has been discovered that CD4(+) T cells function as directly as effector cells and not helper cells in the rejection of corneal allografts. [Hegde S et al. (2005), Transplantation, vol 79: 23-31]. Murine studies have shown increased numbers of neutrophils, macrophage and mast cells in the stroma of corneas undergoing rejection. Macrophages were the main infiltrating cell type followed by T-cells, mast cells and neutrophils. The early chemokine expression in high risk corneal transplantation was the mouse homologue of IL-8 (macrophage inflammatory protein-2) and monocyte chemotactic protein-1 (MCP-1) [Yamagami S et al. (2005), Mol Vis, vol 11, 632-40].

FTY720 (FTY) is a novel immunosuppressive drug that acts by altering lymphocyte trafficking; resulting in peripheral blood lymphopenia and increased lymphocyte counts in lymph nodes. FTY mediates its immune-modulating effects by binding to some of the SIP receptors expressed on lymphocytes. [Bohler T et al. (2005), Transplantation, vol 79: 492-5]. The drug is administered orally and a single oral dose reduced peripheral lymphocyte counts by 30-70%. FTY reduced T-cell subset, CD4(+) cells more than CD8(+) cells. [Bohler et al. (2004), Nephrol Dial Transplant, vol 19: 702-13]. FTY treated mice showed a significant prolongation of orthotopic corneal-graft survival when administered orally. [Zhang et al. (2003), Transplantation, vol 76: 1511-3]. FTY oral treatment also significantly delayed rejection and decreased its severity in a rat-to-mouse model of corneal xenotransplantation [Sedlakova et al. (2005), Transplantation, vol 79, 297-303]. Given the known pathogenesis of allograft rejection combined with the data suggesting that modulating the effects of the S1P signaling can improve corneal graft survival, it is believed that immune moieties that decrease the effective concentration of bioactive lipids, e.g., SPHINGOMAB, will also be useful in treatment of immunologic conditions such as allograft rejection, for example by attenuating the immune response, and thus will likely improve corneal graft survival after PK. The drug may also have the added advantage that in addition to systemic administration, local administration, e.g., via topical periocular or intraocular delivery, may be possible.

Other ocular diseases with an inflammatory or immune component include chronic vitritis, infections, including herpes simplex, herpes zoster and protozoan infections, and ocular histoplasmosis.

### Anterior Segment Diseases Characterized by Scarring

Treatment with an antibody targeted to bioactive lipid also is believed to benefit several conditions characterized by scarring of the anterior portion of the eye. These include the following:

### Trauma

The cornea, as the most anterior structure of the eye, is exposed to various hazards ranging from airborne debris to blunt trauma that can result in mechanical trauma. The cornea and anterior surface of the eye can also be exposed to other forms of trauma from surgery, and chemical, such as acid and alkali, injuries. The results of these types of injuries can be devastating often leading to corneal and conjunctival scarring symblephera formation. In addition corneal neovascularization may ensue. Neutrophils accumulate, their release of leukotrienes, and the presence of interleukin-1 and interleukin-6, serves to recruit successive waves of inflammatory cells [Sotozono et al. (1997), Curr Eye Res, vol 19: 670-676] infiltrate the cornea and release proteolytic enzymes which lead to further damage and break down of corneal tissue and a corneal melt. In addition corneal and conjunctival fibroblasts become activated and invade and leading to collagen deposition and fibrosis. The undesirable effects of excessive inflammation and scarring are promoted by TGF-β. [Saika S et al. (2006), Am J Pathol vol 168, 1848-60]. This process leads to loss of corneal transparency and impaired vision. Reduced inflammation, including decreased neutrophil infiltrates and reduced fibrosis resulted in faster and more complete healing in a murine model of alkali burned corneas [ Ueno et al. (2005), Ophthalmol Vis Sci, vol 46: 4097-106].

### Ocular Cicatricial Pemphigoid (OCP)

OCP is a chronic cicatrizing (scar-forming) autoimmune disease that primarily affects the conjunctiva. The disease is invariably progressive and the prognosis is quite poor. In its final stages conjunctival scarring and the associated keratopathy lead to bilateral blindness. Histologically the conjunctiva shows submucosal scarring and chronic inflammation in which mast cell participation is surprisingly great.[Yao L et al. (2003), Ocul Immunol Inflamm, vol 11: 211-222]. Autoantigens lead to the formation of autoantibodies. The binding of the autoantibody to the autoantigen sets in motion a complex series of events with infiltration of T lymphocytes where CD4 (helper) cells far outnumber CD8 (suppressor) cells. Macrophage and mast cell infiltration also ensue as well as the release of proinflammatory and profibrotic cytokines. Cytokine induced conjunctival fibroblast proliferation and activation results, with resultant subepithelial fibrosis (see examples hereinbelow). Studies have shown a role of TGF-β and IL-1 in conjunctival fibrosis in patients with OCP [Razzaque MS et al. (2004), Invest Ophthalmol Vis Sci, vol 45: 1174-81].

### Stevens Johnson Syndrome (SJS) and Toxic Epidermal Necrolysis TEN)

SJS and TEN are life-threatening adverse reactions to medications. The ocular sequelae of these two related conditions can be severe and involve pathologic changes of the bulbar and palpebral conjunctiva, eyelids and cornea. Drugs and infections are the most common precipitating factors. Chronic eye findings include scarring, symblepharon formation, and cicatrisation of the conjunctiva as a result of the initial inflammatory process. This leads to entropion formation, trichiasis and instability of the tear film. Breakdown of the ocular surface leads to corneal scarring, neovascularization, and in severe cases keratinization. As in OCP subepithelial fibrosis of the conjunctiva occurs. A vigorous autoimmune lymphocyte response to a drug or infection is believed to play a role in development of SJS/TEN. [Harilaos et al. (2005), Erythema Multiforme, Stevens Johnson Syndrome, and Toxic Epidermal Necrolysis, in Cornea 2nd edition. Krachmer, Mannis, Holland eds.Elesevier Mosby Philadelphia]. The infiltrating cell population in SJS includes macrophages, CD4 positive T cells, and CD8 positive T cells. This cell population is similar to those seen in chemical injury. [Kawasaki et al. (2000), J Ophthalmol, vol 84: 1191-3].

### Pteryfgium

Clinically a pterygium appears as a fleshy, vascular mass that occurs in the interpalpebral fissure. The body of the pterygium is a fleshy fibrovascular mass. Active pterygium are characterized by marked vascular engorgement and progressive growth. They are firmly adherent to the globe. In advanced cases the pterygium encroaches onto the cornea and may cause visual loss secondary to loss of corneal transparency within the visual axis or irregular astigmatism. Symptomatically, patients may experience foreign body sensation, tearing and blurred vision. Histopathology demonstrates hyalinization of the subepithelial connective tissue of the substantia propria, increased number of fibroblasts and increased mast cells. [Butrus et al. (1995), Am J Ophthalmol, vol 119: 236-237]. Management of pterygium remains problematic. Surgical excision is often performed however recurrence rates are high. (Krag et al. (1992), Acta Ophthalmol, vol 70: 530]. In order to help lower the recurrence rate of pterygium, various pharmacologic adjuvants have been employed such as Mitomycin-C and daunorubicin. Although these may be helpful, long term data are limited and they can be associated with scleral thinning and corneal melt. Dougherty et al. and Lee et al. [Dougherty et al. (1996), Cornea, vol 15: 537-540 and Lee et al. (2001), Cornea, vol 20: 238-42] were the first to demonstrate that VEGF may play an important role in the development of pterygium and to identify VEGF and nitric oxide in the epithelium of pterygium. These workers hypothesized that these as well as other cytokines are responsible for the fibrovascular ingrowth characteristic of pterygium. The presence of basic FGF and TGF-beta 1 in both primary and recurrent pterygium has been demonstrated [Kira et al. (1998), Graefes Arch Clin Exp Ophthalmol, vol 236: 702-8] and published morphometric and immunohistochemical evidence further supports the notion that angiogenesis may play a role in the formation of pterygium [Marcovich et al (2002), Curr Eye Res, vol 25:17-22]. Other studies have implicated IL-6 and IL-8 as well as VEGF as mediators that may be relevant to pterygium development [Di Girolamo et al. (2006), Invest Ophthalmol Vis Sci, vol 47: 2430-7]. An effective agent against pterygium formation and growth may diminish the need for surgical intervention or reduce recurrence rates.

Other ocular diseases and conditions with a fibrogenesis, fibrosis or scarring component include AMD, diabetic retinopathy, retinopathy of prematurity, sickle cell retinopathy, ischemic retinopathy, retinal venous occlusive disease and contact lens overwear.

In summary, excessive scarring is an underlying component of the pathophysiology of many ocular and non-ocular diseases and conditions. Bioactive lipids like S1P and LPAs play a role in this process and an antibody-related treatment to diminish the concentrations of these agents will likely lead to therapeutic benefit to patients receiving the treatment. In one embodiment, inhibitors of bioactive lipids, particularly monoclonal antibodies directed against S1P and/or LPA, are believed to be useful in modulating surgical and traumatic wound healing responses.

### Anti-S1P and anti-LPA antibodies for the treatment of scleroderma

The compositions and methods disclosed herein will be useful in treating disorders and diseases characterized, at least in part, by aberrant neovascularization, angiogenesis, fibrogenesis, fibrosis, scarring, inflammation, and immune response. One such disease is scleroderma, which is also referred to as systemic sclerosis.

Scleroderma is an autoimmune disease that causes scarring or thickening of the skin, and sometimes involves other areas of the body, including the lungs, heart, and/or kidneys. Scleroderma is characterized by the formation of scar tissue (fibrosis) in the skin and organs of the body, which can lead to thickening and firmness of involved areas, with consequent reduction in function. Today, about 300,000 Americans have scleroderma, according to the Scleroderma Foundation. One-third or less of those affected have widespread disease, while the remaining two-thirds primarily have skin symptoms. When the disease affects the lungs and causing scarring, breathing can become restricted because the lungs can no longer expand as they should. To measure breathing capability, doctors use a device that assesses forced vital capacity (FVC). In people with an FVC of less than 50 percent of the expected reading, the 10-year mortality rate from scleroderma-related lung disease is about 42 percent. One reason the mortality rate is so high is that no effective treatment is currently available.

As described in the examples of this application, existing evidence indicates that S1P and LPA are pro-fibrotic growth factors that can contribute to fibroblast activation, proliferation, and the resulting increased fibroblast activity associated with maladaptive scarring and remodeling. Moreover, potential roles for S1P and LPA in activity of skin and other types of fibroblasts have been demonstrated. For example, it has been shown that LPA stimulates the migration of murine skin fibroblasts (Hama, et al., J Biol Chem. 2004 Apr 23;279(17):17634-9), and human skin fibroblasts express several S1P receptor subtypes (Zhang, et al., Blood. 1999 May 1;93(9):2984-90). In addition to the many direct effects of S1P on fibroblast activity, S1P also may have many potential indirect effects on fibroblast activity. For example, S1P may facilitate the action of other well-known pro-fibrotic factors, such as TGF-β and platelet derived growth factor (PDGF). TGF-β is one of the most widely studied and recognized contributors to fibrosis (Desmouliere, et al., J Cell Biol 122: 103-111, 1993). TGF-β upregulates SphK1 expression and activity leading to increased expression of tissue inhibitors of metalloproteinases 1 (TIMP-1), a protein that inhibits ECM degradation (Yamanaka, et al., J Biol Chem 279: 53994-54001, 2004). Increased expression of TIMP-1 is linked to interstitial fibrosis and diastolic dysfunction in heart failure patients (Heymans, et al., Am J Pathol 166: 15-25, 2005). Conversely, S1P stimulates expression and release of TGF-β (Norata, et al., Circulation 111: 2805-2811, 2005). There is also distinct evidence of crosstalk between S1P and PDGF. S1P directly stimulates expression of PDGF (Usui, et al., J Biol Chem 279: 12300-12311, 2004). In addition, the S1P₁ receptor and the PDGF receptor bind one another and their association is necessary for PDGF activation of downstream signaling which contributes to proliferation and migration of various cell types (Long, et al., Prostaglandins Other Lipid Mediat 80: 74-80, 2006; Baudhuin et al., Faseb J 18: 341-343, 2004). As such, the effects of TGF-β and PDGF on fibrosis may be due in part to crosstalk with the S1P signaling pathway. As such, the compositions and methods of the invention can be used to treat scleroderma, particularly by decreasing the effective in vivo concentration of a particular target lipid, for example, S1P and/or LPA.

Systemic scleroderma is thought to be exacerbated by stimulatory autoantibodies against PDGF receptors (Baroni, et al., N Engl J Med. 2006 v354(25):2667-76), and PDGF receptors are up-regulated in scleroderma fibroblasts in response to TGF-β (Yamakage, et al., J Exp Med. 1992 May 1;175(5):1227-34). Because of the substantial cross-talk among the S1P, PDGF and TGF-β signaling systems, blocking S1P bioactivity with and anti-S1P agent (e.g., an anti-S1P mAb) could indirectly mitigate the pro-sclerotic effects of PDGF and TGF-β. Moreover, treatment with such an anti-S1P agent could benefit scleroderma patients by mitigating the direct effects of S1P on skin and other forms of fibroblasts that contribute to disease progression.

### 3. Methods of Administration

The treatment for diseases and conditions such as the examples given above can be administered by various routes employing different formulations and devices. Suitable pharmaceutically acceptable diluents, carriers, and excipients are well known in the art. One skilled in the art will appreciate that the amounts to be administered for any particular treatment protocol can readily be determined. Suitable amounts might be expected to fall within the range of 10 µg/dose to 10 g/dose, preferably within 10 mg/dose to 1 g/dose.

Drug substances may be administered by techniques known in the art, including but not limited to systemic, subcutaneous, intradermal, mucosal, including by inhalation, and topical administration. The mucosa refers to the epithelial tissue that lines the internal cavities of the body. For example, the mucosa comprises the alimentary canal, including the mouth, esophagus, stomach, intestines, and anus; the respiratory tract, including the nasal passages, trachea, bronchi, and lungs; and the genitalia. For the purpose of this specification, the mucosa will also include the external surface of the eye, i.e. the cornea and conjunctiva. Local administration (as opposed to systemic administration) may be advantageous because this approach can limit potential systemic side effects, but still allow therapeutic effect.

Pharmaceutical compositions used according to the present disclosure include, but are not limited to, solutions, emulsions, and liposome-containing formulations. These compositions may be generated from a variety of components that include, but are not limited to, preformed liquids, self-emulsifying solids and self-emulsifying semisolids.

The pharmaceutical formulations used herein may be prepared according to conventional techniques well known in the pharmaceutical industry. Such techniques include the step of bringing into association the active ingredients with the pharmaceutical carriers) or excipient(s). Preferred carriers include those that are pharmaceutically acceptable, particularly when the composition is intended for therapeutic use in humans. For non-human therapeutic applications (e.g., in the treatment of companion animals, livestock, fish, or poultry), veterinarily acceptable carriers may be employed. In general the formulations are prepared by uniformly and intimately bringing into association the active ingredients with liquid carriers or finely divided solid carriers or both, and then, if necessary, shaping the product.

The compositions disclosed herein may be formulated into any of many possible dosage forms such as, but not limited to, tablets, capsules, liquid syrups, soft gels, suppositories, and enemas. The compositions of the present invention may also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers.

In one embodiment the pharmaceutical compositions may be formulated and used as foams. Pharmaceutical foams include formulations such as, but not limited to, emulsions, microemulsions, creams, jellies and liposomes.

While basically similar in nature these formulations vary in the components and the consistency of the final product. The know-how on the preparation of such compositions and formulations is generally known to those skilled in the pharmaceutical and formulation arts and may be applied to the formulation of the compositions of the present invention.

In one embodiment, an immune-derived moiety can be delivered to the eye via, for example, topical drops or ointment, periocular injection, intracamerally into the anterior chamber or vitreous, via an implanted depot, or systemically by injection or oral administration. The quantity of antibody used can be readily determined by one skilled in the art.

The traditional approaches to delivering therapeutics to the eye include topical application, redistribution into the eye following systemic administration or direct intraocular/periocular injections [Sultana et al. (2006), Current Drug Delivery, vol 3: 207-217; Ghate and Edelhauser (2006), Expert Opinion, vol 3: 275-287 and Kaur and Kanwar (2002), Drug Develop Industrial Pharmacy, vol 28: 473-493]. Anti-S1P, anti-LPA or other anti-bioactive lipid antibody therapeutics would likely be used with any of these approaches although all have certain perceived advantages and disadvantages. Topical drops are convenient, but wash away primarily because of nasolacrimal drainage often delivering less than 5% of the applied drug into the anterior section of the eye and an even smaller fraction of that dose to the posterior segment of the globe. Besides drops, sprays afford another mode for topical administration. A third mode is ophthalmic ointments or emulsions can be used to prolong the contact time of the formulation with the ocular surface although blurring of vision and matting of the eyelids can be troublesome. Such topical approaches are still preferable, since systemic administration of therapeutics to treat ocular disorders exposes the whole body to the potential toxicity of the drug.

Treatment of the posterior segment of the eye is medically important because age-related macular degeneration, diabetic retinopathy, posterior uveitis, and glaucoma are the leading causes of vision loss in the United States and other developed countries. [Myles et al. (2005), Adv Drug Deliv Rev; 57: 2063-79]. The most efficient mode of drug delivery to the posterior segment is intravitreal injection through the pars plana. However, direct injections require a skilled medical practitioner to effect the delivery and can cause treatment-limiting anxiety in many patients. Periocular injections, an approach that includes subconjunctival, retrobulbar, peribulbar and posterior subtenon injections, are somewhat less invasive than intravitreal injections. Repeated and long-term intravitreal injections may cause complications, such as vitreous hemorrhage, retinal detachment, or endophthalmitis.

The anti-bioactive lipid antibody treatment might also be administered using one of the newer ocular delivery systems [Sultana et al. (2006),Current Drug Delivery, vol 3: 207-217 and Ghate and Edelhauser (2006), Expert Opinion, vol 3: 275-287], including sustained or controlled release systems, such as (a) ocular inserts (soluble, erodible, non-erodible or hydrogel-based), corneal shields, eg, collagen-based bandage and contact lenses that provide controlled delivery of drug to the eye, (b) in situ gelling systems that provide ease of administration as drops that get converted to gel form in the eye, thereby providing some sustained effect of drug in the eye, (c) vesicular systems such as liposomes, niosomes/discomes, etc., that offers advantages of targeted delivery, bio-compatibility and freedom from blurring of vision, (d) mucoadhesive systems that provide better retention in the eye, (e) prodrugs (f) penetration enhancers, (g) lyophilized carrier systems, (h) particulates, (i) submicron emulsions, (j) iontophoresis, (k) dendrimers, (1) microspheres including bioadhesive microspheres, (m) nanospheres and other nanoparticles, (n) collasomes and (o) drug delivery systems that combine one or more of the above stated systems to provide an additive, or even synergistic, beneficial effect. Most of these approaches target the anterior segment of the eye and may be beneficial for treating anterior segment disease. However, one or more of these approaches still may be useful affecting bioactive lipid concentrations in the posterior region of the eye because the relatively low molecular weights of the lipids will likely permit considerable movement of the lipid within the eye. In addition, the antibody introduced in the anterior region of the eye may be able to migrate throughout the eye especially if it is manufactured in a lower weight antibody variant such as a Fab fragment. Sustained drug delivery systems for the posterior segment such as those approved or under development (see references, supra) could also be employed.

As previously mentioned, the treatment of disease of the posterior retina, choroids, and macula is medically very important. In this regard, transscleral iontophoresis [Eljarrat-Binstock and Domb (2006), Control Release, 110: 479-89] is an important advance and may offer an effective way to deliver antibodies to the posterior segment of the eye.

Various excipients might also be added to the formulated antibody to improve performance of the therapy, make the therapy more convenient or to clearly ensure that the formulated antibody is used only for its intended, approved purpose. Examples of excipients include chemicals to control pH, antimicrobial agents, preservatives to prevent loss of antibody potency, dyes to identify the formulation for ocular use only, solubilizing agents to increase the concentration of antibody in the formulation, penetration enhancers and the use of agents to adjust isotonicity and/or viscosity. Inhibitors of, e.g., proteases, could be added to prolong the half life of the antibody. In one embodiment, the antibody is delivered to the eye by intravitreal injection in a solution comprising phosphate-buffered saline at a suitable pH for the eye.

The antibody might also be chemically modified to yield a pro-drug that is administered in one of the formulations or devices previously described above. The active form of the antibody is then released by action of an endogenous enzyme. Possible ocular enzymes to be considered in this application are the various cytochrome p450s, aldehyde reductases, ketone reductases, esterases or N-acetyl-β-glucosamidases. Other chemical modifications to the antibody could increase its molecular weight, and as a result, increase the residence time of the antibody in the eye. An example of such a chemical modification is pegylation [Harris and Chess (2003), Nat Rev Drug Discov; 2: 214-21], a process that can be general or specific for a functional group such as disulfide [Shaunak et al. (2006), Nat Chem Biol ; 2:312-3] or a thiol [Doherty et al. (2005), Bioconjug Chem; 16: 1291-8].

### EXAMPLES

The invention will be further described by reference to the following detailed examples. These Examples are in no way to be considered to limit the scope of the invention.

For the data described below, in vitro studies were performed in triplicate and repeated at least three times, and in vivo studies were performed in at least 5 mice. In all studies, statistical analysis was performed using Students T-test or ANOVA using GraphPad software. Where applicable, data are presented as the mean ± SEM with * representing p≤0.05.

### Example 1. SPHINGOMAB Significantly Reduced CNV and Scar Formation in a Murine Model of CNV

Female C57BL6/J mice were subjected to laser-induced rupture of Bruch's membrane and administered either 0.5 µg of Sphingomab or an isotype-matched non-specific (NS) antibody diluted in 2µl of physiological saline. Mice were sacrificed 14 and 28 days after laser rupture.

To induce CNV lesions, the pupils were dilated with ophthalmic tropicamide (0.5%) and phenylephrine (2.5%). A coverslip was placed on the eye. An Oculight GL 532 nm (Iridex Corporation, Mountain View, CA) coupled to a slit lamp set to deliver a 100 msec pulse at 150 mW with a 50 µm spot size was used to rupture Bruch's membrane in three quadrants of the right eye located approximately 50 µm from the optic disc at relative 9, 12 and 3 o'clock positions. The left eye served as an uninjured control in all cases. Any lesion not associated with a vapor bubble or lesions that became confluent were excluded from analysis.

To measure CNV lesion size, choroidal flatmounts of the sclera-choroid-RPE complex were prepared and stained for vasculature (*R. communis agglutinin* I; red) and pericytes (CD140b; green). Digital images were captured using an epifluorescence Zeiss Axioplan 2 with RGB Spot high-resolution digital camera and laser scanning confocal microscope (BioRad MRC 1024, BioRad Corporation, Temecula, CA). For volumetric,analysis, a z-series capture was used and the sum of lesion area throughout the z-series was multiplied by the z thickness (4 µm) to obtain the lesion volume.

To assess collagen deposition, the sclera-choroid-RPE complex was stained with Masson's Trichrome. The sclera-choroid-RPE complex was embedded in paraffin and then serially sectioned at a thickness of 6 microns. Approximately 30 sections per lesion were evaluated. Quantitation of the volume of collagen deposition was calculated in the same manner as described for CNV lesion volume.

Captured digital images are evaluated morphometrically using ImageJ software (Research Services Branch, National Institutes of Health, Bethesda, MD). Figure 1A shows that SPHINGOMAB dramatically attenuates choroidal neovascularization 14 and 28 days after laser-induced rupture of Bruch's membrane. Figure 1B shows that SPHINGOMAB significantly reduces fibrosis associated with CNV lesion formation 28 days after laser-induced rupture of Bruch's membrane.

### Example 2. SPHINGOMAB inhibits neovascularization through multiple mechanisms including inhibition of endothelial cell migration and tube formation.

S1P promotes the migration of human umbilical vein endothelial cells (HUVECs) and, in Matrigel and other assays, the formation of de novo BV formation in vitro [112]; SPHINGOMAB can neutralize these effects of S1P. Experiments were performed as described by Visentin et al. (Cancer Cell 2006 Mar;9(3):225-38). Data in Figure 2A suggest that HUVECs seeded onto GF-reduced Matrigel formed multiple capillary-like structures in the presence of S1P and failed to form capillary-like structures in the absence of S1P or when co-incubated with SPHINGOMAB and S1P. Data in Figure 2B demonstrate the potent ability of 0.1-1µM S1P to stimulate HUVEC migration 2-2.5 fold over non-treated HUVECs, or HUVECs co-incubated with SPHINGOMAB in a Matrigel chemoinvasion assay. Combined, these studies demonstrate that SPHINGOMAB can efficiently mitigate the pro-angiogenic effects of S1P on ECs.

### Example 3. SPHINGOMAB inhibits neovascularization through multiple mechanisms including mitigation of the effects of S1P, VEGF and bFGF in vivo.

Based on in vivo studies showing that S1P increased endothelial capillary growth into subcutaneously implanted Matrigel plugs[54], we speculated that SPHINGOMAB could reduce de novo BV formation in vivo. To investigate this, we employed the in vivo Matrigel Plug assay for neovascularization. In one set of experiments, Matrigel was supplemented with either 1µM S1P, 0.5µg/mL bFGF or 1µg/mL VEGF and then injected I.P. into mice (n=4). After 10 days, the mice were heparinized and injected with the fluorescent lectin, Isolectin B4-FITC, which binds to adhesion molecules expressed by vascular EC that form the growing BVs. The plugs were then excised, frozen in OCT, sectioned and viewed for FITC-stained BVs. Data in Figure 3A suggest that S1P is a more potent stimulator of neovascularization in vivo than bFGF or VEGF[Lee, et al., (1999), Biochem Biophys Res Commun,. vol 264: 743-50], as evidenced by the vast amount of FITC-stained BVs in the plugs containing S1P compared to the plugs containing bFGF or VEGF.

Sections of the plugs were then stained with hemotoxyln & eosin for evaluation of EC infiltration (Figure 3B). The infiltration of ECs is a critical step in neo-vascularization. Plugs containing S1P had a 3-fold increase of EC infiltration in comparison to the Matrigel only plugs. Cell infiltration is presumed to be ECs although we recognize that other cell types such as immune cells may also be stained. Mice systemically administered SPHINGOMAB every 48 hrs (initiated 1 day prior to plug implantation), demonstrated a reduced amount of EC infiltration even when S1P was added to the Matrigel plugs. These results demonstrate the ability of SPHINGOMAB to inhibit EC infiltration in vivo.

Endogenous S1P from the blood and surrounding tissue could supply a wound with pro-angiogenic stimuli. The ability of SPHINGOMAB to reduce endogenous S1P in a wound was investigated. Optimally stimulated plugs (Matrigel supplemented with 0.5µg/mL bFGF or 10mg/mL VEGF) were implanted into mice. Mice received i.p. injections of 25mg/kg SPHINGOMAB or saline every 48 hrs starting 1 day prior to Matrigel implantation. Each treatment group (Matrigel, Matrigel plus GF or Matrigel plus GF and administered SPHINGOMAB) consisted of a minimum of 6 mice. After 10 days, the mice were treated with heparin, injected with Isolectin B4-FITC, the plugs excised, embedded in OCT freezing medium and sectioned. Micro-vascular density was qualitatively accessed by lectin-FITC stained vessels as shown in Figure 3C. BV staining was sporadic in control (untreated) plugs, whereas the plugs containing bFGF or VEGF demonstrated significant evidence of vascularization. The plugs from mice treated with the SPHINGOMAB demonstrated a significant reduction in BV formation compared to the bFGF or VEGF plugs from saline-treated mice. Quantification of stained vessels revealed a 5 to 8.5-fold decrease in neovascularization of VEGF- or bFGF-containing plugs, respectively, from animals treated with SPHINGOMAB in comparison to saline-treated animals (Figure 3C). This evaluation further demonstrates the ability of endogenous serum and tissue S1P to enhance micro-vascularization as well as the ability of SPHINGOMAB to neutralize endogenous S1P's pro-angiogenic effects.

### Example 4. SPHINGOMAB Inhibits Scar Formation in vivo

S1P makes profound contributions to wound healing by activating fibroblast migration, proliferation and collagen production; SPHINGOMAB neutralizes these effects. Several studies using multiple types of fibroblasts confirm S1P's ability to promote wound healing: 1) S1P increased Swiss-3T3 fibroblast proliferation as measured by ³H-thymidine incorporation using standard methods (Figure 4A); 2) S1P promoted the migration of cardiac fibroblasts in a standard scratch wound healing assay. (Figure 4B); 3) S1P promoted collagen expression by cardiac fibroblasts isolated from transgenic mice possessing the collagen la GFP reporter, as indicated by immunofluorescence microscopy (Figure 4C); and 4) S1P induced the differentiation of WI-38 lung fibroblasts into myofibroblasts, cells that are active in scar remodeling, as indicated by increased expression of myofibroblast marker protein, α-smooth muscle actin, using immunoblot analysis (Figure 4D). In each of these assays, SPHINGOMAB neutralized S1P's. It is anticipated that ocular fibroblasts would respond similarly to S1P and SPHINGOMAB. Similarities between cardiovascular disease and neovascular lesions of AMD, including scar remodeling and subsequent, maladaptive fibrous tissue formation, have been noted; [Vine et al. (2005), Ophthalmology,. vol 112: 2076-80 and Seddon and Chen (2004), Int Ophthalmol Clin,. vol 44: 17-39]; thus it is believed that SPHINGOMAB would have effects on ocular neovascularization and scarring similar to those it has demonstrated in cardiovascular systems. Studies at Lpath evaluated the efficacy of SPHINGOMAB to reduce cardiac scar formation after permanent myocardial infarction (MI) via ligation of the left descending coronary artery in mice. Systemic administration of 25mg/kg SPHINGOMAB or saline was initiated 48 hrs after surgery. Antibody administration at 48 hrs was chosen to allow normal, reparative scar formation to occur during the early remodeling phase and permit beneficial, SLOP-stimulated angiogenesis immediately after the MI. Two weeks after the infarct, mice were sacrificed and fibrosis was accessed by Masson's trichrome staining of the cardiac tissue. Animals receiving SPHINGOMAB treatments exhibited almost complete abrogation of perivascular fibrosis (Figure 4E). As a control for any non-specific wound-healing responses, sham animals underwent thoracotomy without coronary artery ligation.

### Example 5: S1P promotes transformation of ocular epithelial cells and fibroblasts into contractile, scar tissue-producing myofibroblasts.

Pathological tissue fibrosis (scar formation) is a primary, contributing factor in a number of ocular disorders, including: age-related macular degeneration, diabetic retinopathy, retinopathy of prematurity, proliferative vitreoretinopathy and consequences of glaucoma surgery.

In many of these disorders, circulating growth factors and chemokines promote the transformation of normal ocular cells into fibrocontractile, scar tissue-producing cells that have been termed "myofibroblasts". Normally, myofibroblasts are responsible for tissue repair as part of the wound healing response following injury. However altered number and function of myofibroblasts are implicated in diseases characterized by pathological scar tissue formation in the liver, skin, lung, kidney, heart and eyes. In the eye, transformation of retinal pigmented epithelial (RPE) cells to a myofibroblast phenotype is linked to formation of fibro-contractile membranes which cause retinal detachment and subsequent vision impairment. In addition, myofibroblast transformation of ocular fibroblasts can result in abnormal scar tissue production after eye injury leading to subsequent vision loss. Although many of the circulating protein factors in the eye that promote myofibroblast formation have been identified, nothing is known regarding the role of lysolipids such as S1P in this process. Therefore, we examined the effects of S1P on myofibroblast transformation of several human ocular cell lines. As shown in Figure 5, S1P stimulates production of α-Smooth muscle actin (α-SMA; a myofibroblast marker) in human retinal pigmented epithelial cells (Figure 5A) and human conjunctiva fibroblasts (Figure 5B). These data demonstrate for the first time, that S1P is among the milieu of circulating chemical factors that promote transformation of ocular epithelial cells and fibroblasts into contractile, scar tissue-producing myofibroblasts which may contribute to retinal detachment, ocular fibrosis and subsequent vision impairment.

In these experiments, the ability of S1P to promote α-SMA expression differed in a concentration dependent manner between the retinal pigmented epithelial cells and conjunctiva fibroblasts. As shown, a significant increase in α-SMA expression was observed at the 0.001 µM concentration in the epithelial cells which then decreased to basal levels at the 10 µM concentration. In contrast, a significant increase in α-SMA expression was observed only at the 10 µM concentration in the conjunctiva fibroblasts. This difference is believed to result from increased S1P receptor expression in the epithelial cells compared to the fibroblasts. We hypothesize that, due to increased S1P receptor expression levels, retinal pigmented epithelial cells are likely more sensitive to S1P at low concentrations. In contrast, at high S1P levels the receptors become sensitized or possibly even internalized leading to decreased stimulation by S1P.

Collagen is one of the primary structural proteins that supports all tissues in the body and is one of the main components of scar tissue. In the non-pathological setting, total collagen content within tissue is maintained via a balance between collagen production by fibroblasts and degradation by certain enzymes. A number of disorders that involve increased levels of scar tissue result, in part, from physiological and molecular processes that inhibit degradation of collagen that is need for scar formation. We hypothesized that the ability of S1P to promote scar tissue formation may result from its ability to inhibit collagen degradation, thereby leading to net increases in scar tissue within organs. Therefore, we examined the effects of S1P on expression of plasminogen activator inhibitor (PAI-1) in human conjunctiva fibroblasts. Increased PAI-1 expression correlates with a decrease in the proteolytic degradation of connective tissue and is upregulated in association with several fibrotic diseases that involve increased scarring. As shown in Figure 5C, S1P stimulates the PAI-1 expression in a dose-dependent manner. These data suggest that, may also promote scar tissue formation by stimulating the expression of proteins that inhibit its degradation, suggesting that S1P functions through multiple mechanistic pathways to promote and maintain pathological scarring associated with ocular diseases.

### Example 6: SPHINGOMAB inhibits inflammatory and immune cell infiltration

Inflammation is the first response in the remodeling process[7]. It is triggered both by ischemia and by cellular damage and results in up-regulation of cytokine expression which stimulates the migration of macrophages and neutrophils to the injured area for phagocytosis of dead cells and to further up-regulate the inflammatory response [Jordan et al.(1999), Cardiovasc Res,. vol 43: 860-78]. Mast cells are also important cellular mediators of the inflammatory response. S1P released from mast cells is responsible for many of the adverse responses seen in experimental animal models of inflammation [Jolly et al (2004), J Exp Med,. vol 199: 959-70 and Jolly et al (2005), Blood,. vol 105: 4736-42].

Based upon the similarities of immune and inflammatory responses in CNV and CVD, the efficacy of SPHINGOMAB to mitigate immune cell infiltration into a wound was evaluated in a murine infarct model as an indication of SPHINGOMAB's potential effects in mitigating these damages during AMD [Vine et al. (2005), Ophthalmology,. vol 112: 2076-80 and Seddon and Chen (2004), Int Ophthalmol Clin,. vol 44: 17-39]. Four days post-MI, macrophage and mast cell infiltration was evaluated using MAC-1 and MCG35 antibodies, respectively, within the area at risk. SPHINGOMAB dramatically attenuated the density of inflammatory macrophages (Figure 6A) and mast cells (Figure 6B) suggesting that SPHINGOMAB may neutralize immune and inflammatory damages during AMD.

### Example 7: SYHINGOMAB is highly specific for S1P

A competitive ELISA demonstrates SPHINGOMAB's specificity for S1P compared to other bioactive lipids. SPHINGOMAB demonstrated no cross-reactivity to sphingosine (SPEH, the immediate metabolic precursor of S1P or lysophosphatidic acid (LPA), an important extracellular signaling molecule that is structurally and functionally similar to S1P. SPHINGOMAB did not recognize other structurally similar lipids and metabolites, including ceramide-1-phosphate (C1P), dihydrosphingosine (DH-SPH), phosphatidyl serine (PS), phosphatidyl ethanolamine (PE), or sphingomyelin (SM). SPHINGOMAB did cross react with dihydrosphingosine-1-phosphate (DH-S1P) and, to a lesser extent, sphingosylphoryl choline (SPC) (Figure 7).

### Example 8: Development of anti-LPA mAbs

The overall objective of these experiments is to generate and develop mAbs specific to LPA to develop an antibody-based therapy for the treatment of LPA related diseases, in particular those diseases which may involve excessive fibrosis. For example, LPA may have some direct fibrogenic effects by stimulating collagen gene expression and proliferation of fibroblasts [Chen, et al. (2006) FEBS Lett. 580(19):4737-45]. Thus, an anti-LPA mAb may be useful in the treatment of fibrosis and diseases characterized by excessive fibroblast activity. These diseases include but are not limited to various ocular disorders, cardiac remodeling and heart failure and scleroderma. Antibodies directed against the bioactive lipid, LPA, that demonstrate good performance characteristics both in *in vitro* assays and *in vivo* would be highly useful in therapeutic and diagnostic applications.

In order to generate monoclonal antibodies (mAbs) against LPA, 80 mice were immunized with a derivative of Lysophosphatidic acid (LPA, 1-acyl-2-lyso-sn-glycero-3-phosphate). The presence of anti-LPA antibodies was determined by analyzing the sera of the immunized mice by ELISA at 3 time points after immunization. The immune response varied greatly between individual mice, both with respect to time of antibody response and levels attained. Overall, a significant immunological response (titer>125,000) was observed in at least half of the mice. Five mice with the highest antibody titer were selected to initiate hybridoma cell line development. After the initial screening of over 2000 hybridoma cells generated from these 5 fusions, a total of 29 anti-LPA secreting hybridoma cell lines exhibited binding to LPA. Of these clones, 24 were further subcloned and characterized in a panel of ELISA assays. From the 14 clones that remained positive, six clones were chosen for further characterization. The isotype of the mAb was IgG1 for most of the antibodies.

In an initial screen, all the mAbs were compared for their binding properties to 12:0 LPA and S1P. Out of 26 mAbs, 5 clones cross-reacted with S1P. Eight anti-LPA mAbs with superior binding to 12:0 and 18:0 LPA were further characterized for their specificity and binding properties.

The mAbs from 6 individual cell lines were fully characterized for their specificity by competition ELISA using a series of analogues and for their potency in a panel of *in vitro* assays (Table 1). The majority of the mAbs exhibited specificity for LPA isoforms. The antibody affinity was estimated to be in the picomolar range. Further testing in animal models will determine whether these mAbs may provide the basis for promising therapies for LPA related diseases.

Using surface plasmon resonance, the affinities and kinetics were measured for 6 mAbs (Table 2). All six mAbs bound LPA with similar *K_{D}* values (ranging from 0.34 to 3.8 pM) and similar kinetic parameters. For many of these interactions, the *k_{d}* was fixed at 1 x 10⁻⁶ s⁻¹ in the fitting program as the complexes dissociated very slowly. Table 1 also depicts the binding of these 6 mAbs to 18:0 and 12:0 LPA bound to the wells of the ELISA plate at increasing concentrations. The concentration of mAb representing 50% of effective concentration (EC₅₀) and the maximal binding (MB) was determined using 18:0 and 12:0 LPA as coating antigen. The EC₅₀ values were generally lower for 18:0 LPA. It is noted that mAb B3 showed a higher binding preference to 18:0 LPA than to 12:0 LPA compared to the other mAbs.

The specificity of the anti-LPA mAbs was evaluated by determining the binding to a set of LPA variants and related biolipids such as distearoyl-phosphatidic acid, lysophosphatidylcholine, S1P, ceramide and ceramide-1-phosphate. The IC₅₀ and cross-reactivity of the 6 selected mAbs plus two additional mAbs (504B58-3F8 and 504B 104) directed against different LPA-related compounds are summarized in Table 3. All mAbs discriminated between 12:0 (lauroyl), 14:0 (myristoyl), 16:0 (palmitoyl), 18:1 (oleoyl), 18:2 (linoleoyl) and 20:4 (arachidonoyl) LPAs (Table 3), while none of them demonstrated cross-reactivity to distearoyl PA and LPC, the immediate metabolic precursor of LPA. Furthermore, the inhibition effect of other lipids tested such as S1P, ceramide and ceramide-1-phosphate were negligible. These findings clearly show that the anti-LPA mAbs did not recognize such structurally similar lipids including the precursor lipids, demonstrating the high specificity of the antibodies to lysophosphatidic acids. The rank order for EC₅₀ was for the unsaturated 18:2> 18:1>20:4 and for the saturated lipids 14:0>16:0>18:0.

Interestingly, competition with 18:1 LPA revealed a different behavior for the 6 mAbs. Amongst the 6 mAbs, 504B3 exhibited the lowest IC₅₀ (50% inhibition of binding). Direct binding of mAb 504B3 to immobilized 18:1 LPA was effectively blocked in presence of added 18:1 LPA with IC₅₀ values of 287 nM. Surprisingly, none of the IC₅₀ values were close to their respective *K_{d}* values for binding to LPA. The IC₅₀ value was at least 100-fold higher than their *K_{d}* (nM range *vs* pM range). 5 mAbs exhibited specificity to 18:1 LPA as shown in the competition assay. 18:1 LPA did not compete with mAb 63 bound to immobilized 18:1 LPA, yet it bound LPA with *K_{d}* values in the picomolar range. Thus, while all six mAbs bound with similar affinities to LPA, five out of six mAbs exhibited effective and specific binding to 18:1 LPA.

### Table 1. Direct binding kinetics.

Increasing amounts of mAbs (up to 40 ng/100 µl reaction mix) were tested for binding to 12:0 LPA or 18:0 LPA (0.1 µM) as coating antigen.

EC₅₀: effective antibody concentration that gives 50 % of the maximum binding.

MB: maximal binding (expressed as OD450).

| | **LPA-C12** | | | | | | | |
|---|---|---|---|---|---|---|---|---|
| | **B3** | **B7** | **B58** | **B58-3F8** | **B104** | **D22-1** | **A63-1** | **B3A6-1** |
| **HMC (nM)** | 2.420 | 0.413 | 0.554 | 0.463 | 0.559 | 1.307 | 0.280 | 0.344 |
| **Max (OD450)** | 0.809 | 1.395 | 1.352 | 1.404 | 1.402 | 0.449 | 1.269 | 1.316 |

| | **LPA-C18** | | | | | | | |
|---|---|---|---|---|---|---|---|---|
| | **B3** | **B7** | **B58** | **B58-3F8** | **B104** | **D22-1** | **A63-1** | **B3A6-1** |
| **HMC (nM)** | 1.067 | 0.274 | 0.245 | 0.187 | 0.313 | 0.176 | 0.298 | 0.469 |
| **Max (OD450)** | 1.264 | 0.973 | 0.847 | 1.000 | 1.016 | 0.353 | 1.302 | 1.027 |

### Table 2. Binding affinity of the anti-LPA mouse mAbs.

LPA was immobilized to the sensor chip at densities ranging 150 resonance units. Dilutions of each mAb were passed over the immobilized LPA and kinetic constants were obtained by nonlinear regression of association/dissociation phases. Errors are given as the standard deviation using at least three determinations in duplicate run. Apparent affinities were determined by K_{D} = *kₐ*/*k_{d}*.
kₐ = Association rate constant in M⁻s⁻¹
k_{d}= Dissociation rate constant in s⁻¹

| **Antibody Molecules** | k_{a (}M⁻¹ s⁻¹) | **k_{d} (s⁻¹)** | **K_{D} (pM)** |
|---|---|---|---|
| **A63** | 4.4 ± 1.0 x 10⁵ | **1 x 10⁻⁶** | **2.3** ± **0.5** |
| **B3** | **7.0 ± 1.5 x 10⁵** | **1 x 10⁻⁶** | **1.4** ± **0.3** |
| **B7** | **6.2** ± **0.1 x 10**⁵ | **1 x 10⁻⁶** | 1.6 ± 0.1 |
| **D22** | **3.0** ± **0.9 x 10⁴** | **1 x 10⁻⁶** | **33** ± **10** |
| **B3A6** | **1.2** ± **0.9 x 10**⁶ | **1.9 ± 0.4 x 10⁻⁵** | **16** ± **1.2** |
| **B58** | **2.9** ± **1.6 x 10⁶** | **1 x 10⁻⁶** | **0.34** ± **0.019** |

### Table 3: Competition of LPA binding and activity.

18:0 LPA was captured on ELISA plates. Each competitor lipid (up to 10 µM) was serially diluted in BSA (1 mg/ml)-PBS and then incubated with the mAbs (3 nM). Mixtures were then transferred to LPA coated wells and the amount of bound antibody was measured with a secondary antibody. Data was normalized to maximum signal (A₄₅₀) and is expressed as percent inhibition. Assays were performed in triplicate for parameter analysis.

| | **14:0 LPA** | | **16:0 LPA** | | **18:1 LPA** | | **18:2 LPA** | | **20:4 LPA** | |
|---|---|---|---|---|---|---|---|---|---|---|
| | **IC₅₀** | **MI** | **IC₅₀** | **MI** | **IC₅₀** | **MI** | **IC₅₀** | **MI** | **IC₅₀** | **MI** |
| | uM | % | uM | % | uM | % | uM | % | uM | % |
| **504B3** | 0.02 | 72.3 | 0.05 | 70.3 | 0.287 | 83 | 0.064 | 72.5 | 0.02 | 67.1 |
| **504B7** | 0.105 | 61.3 | 0.483 | 62.9 | >2.0 | 100 | 1.487 | 100 | 0.161 | 67 |
| **504B58** | 0.179 | 66.7 | 3.061 | >100 | 1.606 | 72.8 | 1.278 | 94.6 | 0.22 | 63.6 |
| **504B58-3F8** | 0.26 | 63.9 | 5.698 | >100 | 1.5 | 79.3 | 1.240 | 92.6 | 0.304 | 79.8 |
| **504B104** | 0.32 | 23.1 | 1.557 | 26.5 | 28.648 | >100 | 1.591 | 36 | 0.32 | 20.1 |
| **504D22-1** | 0.164 | 34.9 | 0.543 | 31 | 1.489 | 47.7 | 0.331 | 31.4 | 0.164 | 29.5 |
| **504A63-1** | 1.147 | 31.9 | 5.994 | 45.7 | --- | --- | --- | --- | 0.119 | 14.5 |
| **504B3A6-1** | 0.108 | 59.9 | 1.151 | 81.1 | 1.897 | 87.6 | --- | --- | 0.131 | 44.9 |

IC₅₀: Half maximum inhibition concentration
MI: Maximum inhibition (% of binding in the absence of inhibitor)
--- : not estimated because of a weak inhibition

### Materials and Methods

### Biolipid reagents:

All biolipids were purchased from Avanti Polar Lipids with identity confirmed by HPLC and mass spectrometry. LPA derivatives were synthesized at the Department of Chemistry (San Diego State University). All other reagents were purchased from Fisher unless otherwise stated.

DLPC: 1-Palmytoyl-2-myristoyl-sn-glycero-3-phosphocholine.

DASA: 1,2-Distearoyl- *sn* -glycero-3-phosphate ;

14:0 LPA: 1-Myristoyl-2-hydroxy- *sn* -glycero-3-phosphate

16:0 LPA: 1-Plmytoyl-2-hydroxy- *sn* -glycero-3-phosphate

18:0 LPA: 1-Stearoyl-2-hydroxy- *sn* -glycero-3-phosphate

18:1 LPA: 1-Oleoyl-2-hydroxy- *sn* -glycero-3-phosphate

18:2 LPA: 1-Linoleoyl-2-hydroxy- *sn* -glycero-3-phosphate

20:4 LPA: 1-Arachidonoyl-2-hydroxy- *sn* -glycero-3-phosphate

S1P: D-erythro-sphingosine-1-phosphate

### Quantitative ELISAs:

Microtiter ELISA plates (Costar, Cat No. 3361) were coated with rabbit anti-mouse IgG, F(ab')₂ fragment specific antibody (Jackson, 315-005-047) diluted in1M Carbonate Buffer (pH 9.5) at 37°C for 1 h. Plates were washed with PBS and blocked with PBSBSA/Tween-20 for 1 hr at 37°C. For the primary incubation, dilutions of non-specific mouse IgG or human IgG, whole molecule (used for calibration curve) and samples to be measured were added to the wells. Plates were washed and incubated with 100 µl per well of HRP conjugated goat anti-mouse (H+L) diluted 1:40,000 (Jackson, cat No 115-035-146) for 1 hr at 37°C. After washing, the enzymatic reaction was detected with tetramethylbenzidine (Sigma, cat No T0440) and stopped by adding 1 M H₂SO₄. The optical density (OD) was measured at 450 nm using a Thermo Multiskan EX. Raw data were transferred to GraphPad software for analysis.

### Direct ELISA:

Microtiter ELISA plates (Costar, Cat No. 3361) were coated with LPA-BSA diluted in 1M Carbonate Buffer (pH 9.5) at 37 °C for 1 h. Plates were washed with PBS (137 mM NaCl, 2.68 mM KCl, 10.1 mM Na₂HPO₄, 1.76 mM KH₂PO₄; pH 7.4) and blocked with PBSBSA/Tween-20 for 1 h at room temperature or overnight at 4°C. The samples to be tested were diluted at 0.4 µg/mL, 0.2 µg/mL, 0.1 µg/mL, 0.05 µg/mL, 0.0125 µg/mL, and 0 µg/mL and 100 µl added to each well. Plates were washed and incubated with 100 µl per well of HRP conjugated goat anti-mouse (1:20,000 dilution) (Jackson, cat No 115-035-003) for 1 h at room temperature. After washing, the enzymatic reaction was detected with tetramethylbenzidine (Sigma, cat No T0440) and stopped by adding 1 M H₂SO₄. The optical density (OD) was measured at 450nm using a Thermo Multiskan EX. Raw data were transferred to GraphPad software for analysis.

### Competition assays:

The specificity of mAbs was tested in ELISA assays. Microtiter plates ELISA plates (Costar, Cat No. 3361) were coated with 18:0 LPA-BSA diluted in 1M Carbonate Buffer (pH 9.5) at 37 °C for 1 h. Plates were washed with PBS (137 mM NaCl, 2.68 mM KCl, 10.1 mM Na₂HPO₄, 1.76 mM KH₂PO₄; pH 7.4) and blocked with PBSBSA/Tween-20 at 37 °C for 1 h or overnight at room temperature. For the primary incubation 0.4 µg/mL anti-LPA mAb and designated amounts of (14:0, 16:0, 18:0, 18:1, 18:2 and 20:4) LPA, DSPA, 18:1 LPC (lysophosphatidylcholine), S1P, ceramide and ceramide-1-phosphate were added to wells of the ELISA plates and incubated at 37 °C for 1 h. Plates were washed and incubated with 100 µl per well of HRP conjugated goat anti-mouse (1:20,000 dilution) (Jackson, cat No 115-035-003) or HRP conjugated goat anti-human(H +L) diluted 1:50,000 (Jackson, cat No109-035-003) at 37 °C for Ih. After washing, the enzymatic reaction was detected with tetramethylbenzidine and stopped by adding 1 M H₂SO₄. The optical density (OD) was measured at 450nm using a Thermo Multiskan EX. Raw data were transferred to GraphPad software for analysis.

### Antibody purification:

Monoclonal antibodies were purified from culture supernatants by passing culture supernatants over protein A/G columns (Pierce, Cat.No 53133) at 0,.5 mL/min. Mobile phases consisted of 1X Pierce IgG binding Buffer (Cat.No 21001) and 0.1 M glycine pH 2.7 (Pierce, Elution Buffer, Cat.No 21004). Antibody collections in 0.1 M glycine were diluted 10 % (v/v) with 1 M Phosphate Buffer, pH 8.0, to neutralize the pH. IgG₁ collections were pooled and dialyzed exhaustively against 1X PBS (Pierce Slide-A-Lyzer Cassette, 3,500 MWCO, Cat.No 66382). Eluates were concentrated using Centricon YM-3(10,000 MWCO Amicon Cat.No 4203) by centrifugation for 1 h at 2,500 rcf. The antibody concentration was determined by quantitative ELISA as described above using a commercial myeloma IgG₁ stock solution as a standard. Heavy chain types of mAbs were determined by ELISA using Monoclonal Antibody Isotyping Kit (Sigma, ISO-2).

### Example 9: Humanized anti-S1P Monoclonal Antibody - SPHINGOMAB

This example describes a particularly preferred humanized monoclonal antibody specifically reactive with S1P termed LT1009 . As compared to the murine anti-S1P antibody from which LT1009 was derived, the humanized form exhibits an S1P binding affinity in the picomolar range, as well as superior stability and *in vivo* efficacy.

As with naturally occurring antibodies, LT1009 includes three complementarity determining regions (each a "CDR") in each of the two light chain polypeptides and each of the two heavy chain polypeptides that comprise each antibody molecule. The amino acid sequences for each of these six CDRs is provided immediately below ("VL" designates the variable region of the immunoglobulin light chain, whereas "VH" designates the variable region of the immunoglobulin heavy chain):
CDR1 VL: ITTTDIDDDMN [SEQ ID NO: 1]
CDR2 VL: EGNILRP [SEQ ID NO: 2]
CDR3 VL: LQSDNLPFT [SEQ ID NO: 3]
CDR1 VH: DHTIH [SEQ ID NO: 4]
CDR3 VH: GGFYGSTIWFDF [SEQ ID NO: 5]
CDR2 VH: AISPRHDITKYNEMFRG [SEQ ID NO: 6]

The nucleotide and amino acid sequences for the heavy and light chain polypeptides of LT1009 are listed immediately below:
LT1009 HC nucleotide sequence [SEQ ID NO: 7]:
LT1009 HC amino acid sequence [SEQ ID NO: 8]:
LT1009 LC nucleotide sequence [SEQ ID NO: 9]:
LT1009 LC amino acid sequence [SEQ ID NO: 10]:

### SEQUENCE LISTING

<110> LPATH, INC.
<120> COMPOSITIONS AND METHODS FOR THE TREATMENT AND PREVENTION OF FIBROTIC, INFLAMMATORY AND NEOVASCULAR CONDITIONS
<130> LPT-3100-PC3
<140>
   <141>
<150> 11/261,935
   <151> 2005-10-28
<160> 10
<170> PatentIn Ver. 3.3
<210> 1
   <211> 11
   <212> PRT
   <213> Artificial Sequence
<220>
   <223> Description of Artificial Sequence: Artificial Humanized Mouse Antibody
<400> 1
   Ile Thr Thr Thr Asp Ile Asp Asp Asp Met Asn
    1               5                   10
<210> 2
   <211> 7
   <212> PRT
   <213> Artificial Sequence
<220>
   <223> Description of Artificial Sequence: Artificial Humanized Mouse Antibody
<400> 2
   Glu Gly Asn Ile Leu Arg Pro
    1               5
<210> 3
   <211> 9
   <212> PRT
   <213> Artificial Sequence
<220>
   <223> Description of Artificial Sequence: Artificial Humanized Mouse Antibody
<400> 3
   Leu Gln Ser Asp Asn Leu Pro Phe Thr
    1               5
<210> 4
   <211> 5
   <212> PRT
   <213> Artificial Sequence
<220>
   <223> Description of Artificial Sequence: Artificial Humanized Mouse Antibody
<400> 4
   Asp His Thr Ile His
    1               5
<210> 5
   <211> 12
   <212> PRT
   <213> Artificial Sequence
<220>
   <223> Description of Artificial Sequence: Artificial Humanized Mouse Antibody
<400> 5
   Gly Gly Phe Tyr Gly Ser Thr Ile Trp Phe Asp Phe
    1               5                   10
<210> 6
   <211> 17
   <212> PRT
   <213> Artificial Sequence
<220>
   <223> Description of Artificial Sequence: Artificial Humanized Mouse Antibody
<400> 6
   Ala Ile Ser Pro Arg His Asp Ile Thr Lys Tyr Asn Glu Met Phe Arg
    1               5                   10                  15
   Gly
<210> 7
   <211> 2034
   <212> DNA
   <213> Artificial Sequence
<220>
   <223> Description of Artificial Sequence: Artificial Humanized Mouse Antibody
<400> 7
   aagcttgccg ccaccatgga atggagctgg gtgttcctgt tctttctgtc cgtgaccaca 60
   ggcgtgcatt ctgaggtgca gctggtgcag tctggagcag aggtgaaaaa gcccggggag 120
   tctctgaaga tctcctgtca gagttttgga tacatcttta tcgaccatac tattcactgg 180
   atgcgccaga tgcccgggca aggcctggag tggatggggg ctatttctcc cagacatgat 240
   attactaaat acaatgagat gttcaggggc caggtcacca tctcagccga caagtccagc 300
   agcaccgcct acttgcagtg gagcagcctg aaggcctcgg acaccgccat gtatttctgt 360
   gcgagagggg ggttctacgg tagtactatc tggtttgact tttggggcca agggacaatg 420
   gtcaccgtct cttcagcctc caccaagggc ccatcggtct tccccctggc accctcctcc 480
   aagagcacct ctgggggcac agcggccctg ggctgcctgg tcaaggacta cttccccgaa 540
   ccggtgacgg tgtcgtggaa ctcaggcgcc ctgaccagcg gcgtgcacac cttcccggct 600
   gtcctacagt cctcaggact ctactccctc agcagcgtgg tgaccgtgcc ctccagcagc 660
   ttgggcaccc agacctacat ctgcaacgtg aatcacaagc ccagcaacac caaggtggac 720
   aagagagttg gtgagaggcc agcacaggga gggagggtgt ctgctggaag ccaggctcag 780
   cgctcctgcc tggacgcatc ccggctatgc agtcccagtc cagggcagca aggcaggccc 840
   cgtctgcctc ttcacccgga ggcctctgcc cgccccactc atgctcaggg agagggtctt 900
   ctggcttttt ccccaggctc tgggcaggca caggctaggt gcccctaacc caggccctgc 960
   acacaaaggg gcaggtgctg ggctcagacc tgccaagagc catatccggg aggaccctgc 1020
   ccctgaccta agcccacccc aaaggccaaa ctctccactc cctcagctcg gacaccttct 1080
   ctcctcccag attccagtaa ctcccaatct tctctctgca gagcccaaat cttgtgacaa 1140
   aactcacaca tgcccaccgt gcccaggtaa gccagcccag gcctcgccct ccagctcaag 1200
   gcgggacagg tgccctagag tagcctgcat ccagggacag gccccagccg ggtgctgaca 1260
   cgtccacctc catctcttcc tcagcacctg aactcctggg gggaccgtca gtcttcctct 1320
   tccccccaaa acccaaggac accctcatga tctcccggac ccctgaggtc acatgcgtgg 1380
   tggtggacgt gagccacgaa gaccctgagg tcaagttcaa ctggtacgtg gacggcgtgg 1440
   aggtgcataa tgccaagaca aagccgcggg aggagcagta caacagcacg taccgtgtgg 1500
   tcagcgtcct caccgtcctg caccaggact ggctgaatgg caaggagtac aagtgcaagg 1560
   tctccaacaa agccctccca gcccccatcg agaaaaccat ctccaaagcc aaaggtggga 1620
   cccgtggggt gcgagggcca catggacaga ggccggctcg gcccaccctc tgccctgaga 1680
   gtgaccgctg taccaacctc tgtccctaca gggcagcccc gagaaccaca ggtgtacacc 1740
   ctgcccccat cccgggagga gatgaccaag aaccaggtca gcctgacctg cctggtcaaa 1800
   ggcttctatc ccagcgacat cgccgtggag tgggagagca atgggcagcc ggagaacaac 1860
   tacaagacca cgcctcccgt gctggactcc gacggctcct tcttcctcta tagcaagctc 1920
   accgtggaca agagcaggtg gcagcagggg aacgtcttct catgctccgt gatgcatgag 1980
   gctctgcaca accactacac gcagaagagc ctctccctgt ctccgggtaa atag       2034
<210> 8 .
   <211> 455
   <212> PRT
   <213> Artificial Sequence
<220>
   <223> Description of Artificial Sequence: Artificial Humanized Mouse Antibody
<400> 8
   Met Glu Trp Ser Trp Val Phe Leu Phe Phe Leu Ser Val Thr Thr Gly
    1               5                  10                  15
   Val His Ser Glu Val Gln Leu Val Gln Ser Gly Ala Glu Val Lys Lys
               20                  25                  30
   Pro Gly Glu Ser Leu Lys Ile Ser Cys Gln Ser Phe Gly Tyr Ile Phe
           35                  40                  45
   Ile Asp His Thr Ile His Trp Met Arg Gln Met Pro Gly Gln Gly Leu
   50 55 60
   Glu Trp Met Gly Ala Ile Ser Pro Arg His Asp Ile Thr Lys Tyr Asn
   65 70 75 80
   Glu Met Phe Arg Gly Gln Val Thr Ile Ser Ala Asp Lys Ser Ser Ser
   85 90 95
   Thr Ala Tyr Leu Gln Trp Ser Ser Leu Lys Ala Ser Asp Thr Ala Met
   100 105 110
   Tyr Phe Cys Ala Arg Gly Gly Phe Tyr Gly Ser Thr Ile Trp Phe Asp
   115 120 125
   Phe Trp Gly Gln Gly Thr Met Val Thr Val Ser Ser Ala Ser Thr Lys
   130 135 140
   Gly Pro Ser Val Phe Pro Leu Ala Pro Ser Ser Lys Ser Thr Ser Gly
   145 150 155 160
   Gly Thr Ala Ala Leu Gly Cys Leu Val Lys Asp Tyr Phe Pro Glu Pro
   165 170 175
   Val Thr Val Ser Trp Asn Ser Gly Ala Leu Thr Ser Gly Val His Thr
   180 185 190
   Phe Pro Ala Val Leu Gln Ser Ser Gly Leu Tyr Ser Leu Ser Ser Val
   195 200 205
   Val Thr Val Pro Ser Ser Ser Leu Gly Thr Gln Thr Tyr Ile Cys Asn
   210 215 220
   Val Asn His Lys Pro Ser Asn Thr Lys Val Asp Lys Arg Val Ala Pro
   225 230 235 240
   Glu Leu Leu Gly Gly Pro Ser Val Phe Leu Phe Pro Pro Lys Pro Lys
   245 250 255
   Asp Thr Leu Met Ile Ser Arg Thr Pro Glu Val Thr Cys Val Val Val
   260 265 270
   Asp Val Ser His Glu Asp Pro Glu Val Lys Phe Asn Trp Tyr Val Asp
   275 280 285
   Gly Val Glu Val His Asn Ala Lys Thr Lys Pro Arg Glu Glu Gln Tyr
   290 295 300
   Asn Ser Thr Tyr Arg Val Val Ser Val Leu Thr Val Leu His Gln Asp
   305 310 315 320
   Trp Leu Asn Gly Lys Glu Tyr Lys Cys Lys Val Ser Asn Lys Ala Leu
   325 330 335
   Pro Ala Pro Ile Glu Lys Thr Ile Ser Lys Ala Lys Gly Gln Pro Arg
   340 345 350
   Glu Pro Gln Val Tyr Thr Leu Pro Pro Ser Arg Glu Glu Met Thr Lys
           355                 360                 365
   Asn Gln Val Ser Leu Thr Cys Leu Val Lys Gly Phe Tyr Pro Ser Asp
       370                 375                 380
   Ile Ala Val Glu Trp Glu Ser Asn Gly Gln Pro Glu Asn Asn Tyr Lys
   385                 390                 395                 400
   Thr Thr Pro Pro Val Leu Asp Ser Asp Gly Ser Phe Phe Leu Tyr Ser
                   405                 410                 415
   Lys Leu Thr Val Asp Lys Ser Arg Trp Gln Gln Gly Asn Val Phe Ser
               420                 425                 430
   Cys Ser Val Met His Glu Ala Leu His Asn His Tyr Thr Gln Lys Ser
           435                 440                 445
   Leu Ser Leu Ser Pro Gly Lys
       450                 455
<210> 9
   <211> 720
   <212> DNA
   <213> Artificial Sequence
<220>
   <223> Description of Artificial Sequence: Artificial Humanized Mouse Antibody
<400> 9
   aagcttgccg ccaccatgtc tgtgcctacc caggtgctgg gactgctgct gctgtggctg 60
   acagacgccc gctgtgaaac gacagtgacg cagtctccat ccttcctgtc tgcatctgta 120
   ggagacagag tcaccatcac ttgcataacc accactgata ttgatgatga tatgaactgg 180
   ttccagcagg aaccagggaa agcccctaag ctcctgatct ccgaaggcaa tattcttcgt 240
   cctggggtcc catcaagatt cagcagcagt ggatatggca cagatttcac tctcaccatc 300
   agcaaattgc agcctgaaga ttttgcaact tattactgtt tgcagagtga taacttacca 360
   ttcactttcg gccaagggac caagctggag atcaaacgta cggtggctgc accatctgtc 420
   ttcatcttcc cgccatctga tgagcagttg aaatctggaa ctgcctctgt tgtgtgcctg 480
   ctgaataact tctatcccag agaggccaaa gtacagtgga aggtggataa cgccctccaa 540
   tcgggtaact cccaggagag tgtcacagag caggacagca aggacagcac ctacagcctc 600
   agcagcaccc tgacgctgag caaagcagac tacgagaaac acaaagtcta cgcctgcgaa 660
   gtcacccatc agggcctgag ctcgcccgtc acaaagagct tcaacagggg agagtgttag 720
<210> 10
   <211> 234
   <212> PRT
   <213> Artificial Sequence
<220>
   <223> Description of Artificial Sequence: Artificial Humanized Mouse Antibody
<400> 10
   Met Ser Val Pro Thr Gln Val Leu Gly Leu Leu Leu Leu Trp Leu Thr
    1               5                  10                  15
   Asp Ala Arg Cys Glu Thr Thr Val Thr Gln Ser Pro Ser Phe Leu Ser
               20                  25                      30
   Ala Ser Val Gly Asp Arg Val Thr Ile Thr Cys Ile Thr Thr Thr Asp
           35                  40                      45
   Ile Asp Asp Asp Met Asn Trp Phe Gln Gln Glu Pro Gly Lys Ala Pro
       50                  55                      60
   Lys Leu Leu Ile Ser Glu Gly Asn Ile Leu Arg Pro Gly Val Pro Ser
   65                  70                      75                  80
   Arg Phe Ser Ser Ser Gly Tyr Gly Thr Asp Phe Thr Leu Thr Ile Ser
                   85                      90                  95
   Lys Leu Gln Pro Glu Asp Phe Ala Thr Tyr Tyr Cys Leu Gln Ser Asp
               100                 105                     110
   Asn Leu Pro Phe Thr Phe Gly Gln Gly Thr Lys Leu Glu Ile Lys Arg
           115                 120                     125
   Thr Val Ala Ala Pro Ser Val Phe Ile Phe Pro Pro Ser Asp Glu Gln
       130                 135                     140
   Leu Lys Ser Gly Thr Ala Ser Val Val Cys Leu Leu Asn Asn Phe Tyr
   145                 150                     155                 160
   Pro Arg Glu Ala Lys Val Gln Trp Lys Val Asp Asn Ala Leu Gln Ser
                   165                     170                 175
   Gly Asn Ser Gln Glu Ser Val Thr Glu Gln Asp Ser Lys Asp Ser Thr
               180                 185                     190
   Tyr Ser Leu Ser Ser Thr Leu Thr Leu Ser Lys Ala Asp Tyr Glu Lys
           195                 200                     205
   His Lys Val Tyr Ala Cys Glu Val Thr His Gln Gly Leu Ser Ser Pro
       210                 215                     220
   Val Thr Lys Ser Phe Asn Arg Gly Glu Cys
   225                 230

## Claims

1. A humanized monoclonal antibody, or a fragment, variant, or derivative thereof that binds and neutralizes a bioactive lipid or bioactive lipid precursor or metabolite, wherein the bioactive lipid, bioactive lipid precursor or metabolite is sphingosine-1-phosphate (S1P) or an S1P precursor or metabolite, for use in a method for decreasing the effective concentration of said bioactive lipid in an eye of an animal in order to
(i) treat or prevent aberrant fibrogenesis, fibrosis, or scarring in the eye,
(ii) modulate surgical or traumatic wound healing responses in the eye,
(iii) decrease or prevent inflammation in the eye,
(iv) decrease or prevent aberrant neovascularization of the eye,
(v) attenuate an ocular immune response in the eye, or
(vi) decrease the effective ocular concentration or activity of the bioactive lipid, bioactive lipid precursor or metabolite in order to treat or prevent an ocular disease or condition that is characterized, at least in part, by aberrant fibrogenesis, fibrosis, scarring, inflammation, aberrant neovascularization, or an immune response in the eye.

2. A humanized monoclonal antibody, or a fragment, variant, or derivative thereof as defined in claim 1 for use according to claim 1 wherein the animal is a human.

3. A humanized monoclonal antibody, or a fragment, variant, or derivative thereof as defined in claim 1 for use according to claim 1 wherein (i) the ocular disease or condition characterized, at least in part, by aberrant neovascularization is selected from the group consisting of age-related macular degeneration, diabetic retinopathy, retinopathy of prematurity, corneal graft rejection, neovascular glaucoma, contact lens overwear, infections of the cornea, including herpes simplex infection, herpes zoster infection and protozoan infection; pterygium, ischemic retinopathy, retinal venous occlusive disease, infectious uveitis, chronic retinal detachment, laser injury, sickle cell retinopathy, venous occlusive disease, choroidal neovascularization, retinal angiomatous proliferation, and idiopathic polypoidal choroidal vasculopathy; or (ii) the ocular disease or condition characterized, at least in part, by aberrant fibrogenesis, fibrosis, or scarring is selected from the group consisting of age-related macular degeneration, diabetic retinopathy, retinopathy of prematurity, sickle cell retinopathy, ischemic retinopathies, retinal venous occlusive disease, macular pucker, cellophane retinopathy, ERM formation, contact lens overwear, tractional retinal detachment, proliferative vitreoretinopathy, traumatic injury, ocular cicatricial pemphigoid, Stevens Johnson Syndrome, toxic epidermal necrolysis, pterygium, and consequences of ocular surgery, including refractive surgery, vitrectomy and glaucoma surgery.

4. A humanized monoclonal antibody, or a fragment, variant, or derivative thereof as defined in claim 1 for use according to claim 1 wherein the ocular disease or condition is an inflammatory or immunologic condition, optionally an inflammatory or immunologic condition selected from the group consisting of age-related macular degeneration, uveitis, vitritis, infections, including herpes simplex infection, herpes zoster infection and protozoan infection; corneal graft rejection and ocular histoplasmosis.

5. A humanized monoclonal antibody, or a fragment, variant, or derivative thereof as defined in claim 1 for use according to any of claims 1-4 wherein the humanized monoclonal antibody, or a fragment, variant, or derivative of a monoclonal antibody is intended to be administered systemically, topically, by intravitreal or periocular injection, iontophoresis, spray or drops, or as part of an in situ gel, ocular insert, corneal shield or contact lens, liposome, niosome/ discome, mucoadhesive system, lyophilized carrier system, particulate, submicron emulsion, dendrimer, microsphere, nanosphere, or collasome, or combination thereof.

6. A humanized monoclonal antibody, or a fragment, variant, or derivative thereof as defined in any one of claim 1-5 for use according to any one of claims 1-5 wherein the humanized monoclonal antibody, or a fragment, variant, or derivative of a monoclonal antibody is modified, unmodified, or provided as a prodrug, with or without enhancers and/or penetration enhancers.

7. A humanized monoclonal antibody, or a fragment, variant, or derivative thereof as defined in any one of claim 1-6 for use according to any one of claims 1-6,wherein the humanized monoclonal antibody, or a fragment, variant, or derivative of a monoclonal antibody is combined with a pharmaceutically acceptable carrier and/or phosphate-buffered saline.

## Patentansprüche

1. Humanisierter monoklonaler Antikörper oder ein Fragment, eine Variante oder ein Derivat davon, der ein bioaktives Lipid oder einen bioaktiven Lipidvorläufer oder Metaboliten bindet und neutralisiert, wobei das bioaktive Lipid, der bioaktive Lipidvorläufer oder Metabolit ein Sphingosin-1-phosphat (S1P) oder ein S1P-Vorläufer oder -Metabolit ist, zur Verwendung in einem Verfahren zur Verringerung der wirksamen Konzentration des bioaktiven Lipids in einem Auge eines Tieres, um
(i) aberrante Fibrogenese, Fibrose oder Vernarbung in dem Auge zu behandeln oder zu verhindern
(ii) operative oder traumatische Wundheilungsprozesse in dem Auge zu modulieren,
(iii) Entzündung in dem Auge zu verringern oder zu verhindern
(iv) aberrante Neovaskularisierung des Auges zu behandeln oder zu verhindern,
(v) eine okulare Immunantwort in dem Auge abzuschwächen, oder
(vi) die wirksame okulare Konzentration oder Aktivität des bioaktiven Lipids, bioaktiven Lipidvorläufers oder Metaboliten zu verringern, um eine zumindest teilweise durch aberrante Fibrogenese, Fibrose, Vernarbung, Entzündung, aberannte Neovaskulisierung oder eine Immunantwort in dem Auge gekennzeichnete Krankheit oder Zustand des Auges zu behandeln oder zu verhindern.

2. Humanisierter monoklonaler Antikörper oder ein Fragment, eine Variante oder ein Derivat davon, wie in Anspruch 1 definiert, zur Verwendung gemäß Anspruch 1, wobei das Tier ein Mensch ist.

3. Humanisierter monoklonaler Antikörper oder ein Fragment, eine Variante oder ein Derivat davon, wie in Anspruch 1 definiert, zur Verwendung gemäß Anspruch 1, wobei (i) die zumindest teilweise durch aberrante Fibrogenese in dem Auge gekennzeichnete Krankheit oder der Zustand des Auges aus der Gruppe ausgewählt wird, die aus altersbedingter Makuladegeneration, diabetischer Retinopathie, Premature Retinopathie, Hornhauttransplant-Abstoßung, Neovaskulärem Glaukom, zu langem Tragen von Kontaktlinsen, Infektionen der Hornhaut einschließlich Herpes-Simplex-Infektion, Herpes-Zoster Infektion und Protozoen-Infektion; Pterygium, Ischämische Retinopathie, krankhaftem Venenverschluss der Netzhaut, Infektiöse Uveitis, Chronische Netzhautablösung, Sichelzellenretinopathie, krankhaftem Venenverschluss, Choroidale Neovaskulasierung, Retinale Angiomatöse Proliferation und Idiopatische Polypiodale Choroidale Vaskulopathie besteht, oder (ii) die zumindest teilweise durch aberrante Fibrogenese, Fibrose oder Vernarbung in dem Auge gekennzeichnete Krankheit oder der Zustand des Auges aus der Gruppe ausgewählt wird, die aus altersbedingter Makuladegeneration, diabetischer Retinopathie, Homhauttransplant-Abstoßung, Sichelzellenretinopathie, Ischämischen Retinopathien, krankhaftem Venenverschluss der Netzhaut, Makula Pucker, Zellophan Retinopathie, ERM Bildung, zu langem Tragen von Kontaktlinsen, Netzhautablösung durch Zug, Proliferatorische Vitreoretinophatie, Traumatischer Verletzung, Zikatriziales Pemphigoid des Auges, Stevens Johnson Syndrom, Toxische Epidermale Nekrolyse, Pterygium und Folgen von Augenoperationen einschließlich operativer Sehfehlerkorrektur, Vitrektomie und Glaukom-Operationen besteht.

4. Humanisierter monoklonaler Antikörper oder ein Fragment, eine Variante oder ein Derivat davon, wie in Anspruch 1 definiert, zur Verwendung gemäß Anspruch 1, wobei die Krankheit oder der Zustand des Auges ein entzündlicher oder immunologischer Zustand ist, gegebenenfalls ein entzündlicher oder immunologischer Zustand, der aus der Gruppe ausgewählt wird, die aus altersbedingter Makuladegeneration, Uveitis, Vitritis, Infektionen, einschließlich Herpes-Simplex-Infektion, Herpes-Zoster Infektion und Protozoen-Infektion, Hornhauttransplant-Abstoßung und Augen-Histoplasmose besteht.

5. Humanisierter monoklonaler Antikörper oder ein Fragment, eine Variante oder ein Derivat davon, wie in einem von Anspruch 1-4 definiert, zur Verwendung gemäß einem der Ansprüche 1-4, wobei der monoklonale Antikörper oder ein Fragment, eine Variante oder ein Derivat eines monoklonalen Antikörpers vorgesehen ist, systemisch, topisch, durch intravitreale oder periokulare Injektion, Iontophorese, Spray oder Tropfen oder als Teil eines in situ Geles, Augeninserts, Hornhautschield oder Kontaktlinsen, Liposomen, Niosome/Discomen, eines mukoadhesiven Systems, eines lyophilisierten Trägersystems, partikulär, Submikrometer-Emulsion, Dendrimers, einer Mikrokugel, eine Nanokugel, eines Collasomes oder Kombinationen davon verabreicht zu werden.

6. Humanisierter monoklonaler Antikörper oder ein Fragment, eine Variante oder ein Derivat davon, wie in einem von Anspruch 1-5 definiert, zur Verwendung gemäß einem der Ansprüche 1-5, wobei der monoklonale Antikörper oder ein Fragment, eine Variante oder ein Derivat eines monoklonalen Antikörpers modifiziert, unmodifiziert ist oder bereitgestellt als Pro-Drug, mit oder ohne Verstärker und/oder Penetrationsverstärker.

7. Humanisierter monoklonaler Antikörper oder ein Fragment, eine Variante oder ein Derivat davon, wie in einem von Anspruch 1-6 definiert, zur Verwendung gemäß einem der Ansprüche 1-6, wobei der monoklonale Antikörper oder ein Fragment, eine Variante oder ein Derivat davon mit einem pharmazeutisch annehmbaren Träger und/oder Phosphat gepuffertem Salin kombiniert wird.

## Revendications

1. Anticorps monoclonal humanisé, ou fragment, variante, ou dérivé de celui-ci qui se lie et neutralise un lipide bioactif ou un précurseur ou un métabolite de lipide bioactif, dans lequel le lipide bioactif, le précurseur ou le métabolite de lipide bioactif est le sphingosine-1-phosphate (S1P) ou un précurseur ou métabolite de S1P, à utiliser dans un procédé pour diminuer la concentration efficace dudit lipide bioactif dans un oeil d'un animal afin de
(i) traiter ou empêcher une fibrogénèse aberrante, une fibrose, ou une lésion dans l'oeil,
(ii) moduler les réponses de cicatrisation de plaies chirurgicales ou traumatiques dans l'oeil,
(iii) diminuer ou empêcher une inflammation dans l'oeil,
(iv) diminuer ou empêcher une néovascularisation aberrante dans l'oeil,
(v) atténuer une réponse immunitaire oculaire dans l'oeil, ou
(vi) diminuer la concentration oculaire efficace ou l'activité du lipide bioactif, du précurseur ou du métabolite de lipide bioactif afin de traiter ou d'empêcher une maladie ou une condition oculaire qui est caractérisée, au moins en partie, par une fibrogénèse aberrante, une fibrose, une lésion, une inflammation, une néovascularisation aberrante, ou une réponse immunitaire dans l'oeil.

2. Anticorps monoclonal humanisé, ou fragment, variante, ou dérivé de celui-ci tel que défini à la revendication 1 à utiliser selon la revendication 1, dans lequel l'animal est un humain.

3. Anticorps monoclonal humanisé, ou fragment, variante, ou dérivé de celui-ci tel que défini à la revendication 1 à utiliser selon la revendication 1, dans lequel (i) la maladie ou la condition oculaire caractérisée, au moins en partie, par une néovascularisation aberrante est sélectionnée parmi le groupe constitué de la dégénérescence maculaire liée à l'âge, la rétinopathie diabétique, la fibroplasie rétrocristallinienne, le rejet de greffe de cornée, le glaucome néovasculaire, l'excès de port de lentilles de contact, des infections de la cornée, y compris l'infection à l'herpès simplex, le zona et une infection causée par des protozoaires ; le ptérygion, la rétinopathie ischémique, la maladie de l'occlusion veineuse rétinienne, l'uvéite infectieuse, le décollement de rétine chronique, une blessure au laser, la rétinopathie drépanocytaire, la maladie de l'occlusion veineuse, la néovascularisation choroïdienne, la prolifération angiomateuse rétinienne, et la vasculopathie choroïdienne polypoïdale idiopathique ; ou (ii) la maladie ou la condition oculaire caractérisée, au moins en partie, par une fibrogénèse aberrante, une fibrose, ou une lésion est sélectionnée parmi le groupe constitué de la dégénérescence maculaire liée à l'âge, la rétinopathie diabétique, la fibroplasie rétrocristallinienne, la rétinopathie drépanocytaire, les rétinopathies ischémiques, la maladie de l'occlusion veineuse rétinienne, une membrane épirétinienne, la fibrose maculaire prérétinienne, la formation de MER, l'excès de port de lentilles de contact, le décollement de la rétine par traction, la vitréorétinopathie proliférante, une blessure traumatique, un pemphigoïde cicatriciel oculaire, le syndrome de Stevens-Johnson, le syndrome de Lyell, le ptérygion, et des conséquences d'une chirurgie oculaire, y compris une chirurgie réfractive, une vitrectomie et une chirurgie du glaucome.

4. Anticorps monoclonal humanisé, ou fragment, variante, ou dérivé de celui-ci tel que défini à la revendication 1 à utiliser selon la revendication 1, dans lequel la maladie ou la condition oculaire est une condition inflammatoire ou immunologique, éventuellement une condition inflammatoire ou immunologique sélectionnée parmi le groupe constitué de la dégénérescence maculaire liée à l'âge, l'uvéite, l'hyalite, des infections, y compris l'infection à l'herpès simplex, le zona et une infection causée par des protozoaires ; le rejet de greffe de cornée et l'histoplasmose oculaire.

5. Anticorps monoclonal humanisé, ou fragment, variante, ou dérivé de celui-ci tel que défini à la revendication 1 à utiliser selon l'une quelconque des revendications 1 à 4, dans lequel l'anticorps monoclonal humanisé, ou fragment, variante ou dérivé d'un anticorps monoclonal est prévu pour être administré de façon systémique, topique, par injection intravitréale ou périoculaire, par iontophorèse, pulvérisateur ou gouttes, ou comme partie d'un gel in situ, d'un dispositif oculaire, d'un bouclier cornéen ou d'une lentille de contact, comme liposome, niosome/discome, système mucoadhésif, système de support lyophilisé, matière particulaire, émulsion submicronique, dendrimère, microsphère, nanosphère, ou collasome, ou des combinaisons de ceux-ci.

6. Anticorps monoclonal humanisé, ou fragment, variante, ou dérivé de celui-ci tel que défini dans l'une quelconque des revendications 1 à 5 à utiliser selon l'une quelconque des revendications 1 à 5, dans lequel l'anticorps monoclonal humanisé, ou fragment, variante ou dérivé d'un anticorps monoclonal est modifié, non modifié, ou prévu comme promédicament, avec ou sans activateurs et/ou activateurs de pénétration.

7. Anticorps monoclonal humanisé, ou fragment, variante, ou dérivé de celui-ci tel que défini dans l'une quelconque des revendications 1 à 6 à utiliser selon l'une quelconque des revendications 1 à 6, dans lequel l'anticorps monoclonal humanisé, ou fragment, variante ou dérivé d'un anticorps monoclonal est combiné à un support pharmaceutiquement acceptable et/ou à une solution saline tamponnée au phosphate.
